(19) Europäisches Patentamt
European Patent Office
Office européen des brevets

(11) EP 4 613 785 A1

(12) # EUROPEAN PATENT APPLICATION
published in accordance with Art. 153(4) EPC

(43) Date of publication:
**10.09.2025 Bulletin 2025/37**

(21) Application number: **23885749.4**

(22) Date of filing: **31.10.2023**

(51) International Patent Classification (IPC):
***C08F 20/18*** *(2006.01)* ***C07C 67/03*** *(2006.01)*
***C07C 67/62*** *(2006.01)* ***C07C 69/54*** *(2006.01)*

(52) Cooperative Patent Classification (CPC):
**C07C 67/03; C07C 67/62; C07C 69/54; C08F 20/18**

(86) International application number:
**PCT/JP2023/039202**

(87) International publication number:
**WO 2024/095995 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.10.2022 JP 2022174445**
**31.10.2022 JP 2022174450**
**31.10.2022 JP 2022174455**

(71) Applicant: **Mitsubishi Chemical Corporation**
**Tokyo 100-8251 (JP)**

(72) Inventor: **AIZAWA, Ryo**
**Tokyo 100-8251 (JP)**

(74) Representative: **Hoffmann Eitle**
**Patent- und Rechtsanwälte PartmbB**
**Arabellastraße 30**
**81925 München (DE)**

(54) **ESTER-COMPOUND-CONTAINING COMPOSITION AND METHOD FOR PRODUCING SAME, POLYMERIZABLE COMPOSITION, AND (METH)ACRYLIC POLYMER AND METHOD FOR PRODUCING SAME**

(57) An object is to provide an ester-compound-containing composition and a method for producing the same, that can be produced efficiently and has excellent storage stability, a polymerizable composition using the ester-compound-containing composition, and a (meth) acrylic polymer and a method for producing the same. The ester-compound-containing composition contains an ester compound (I) that is one or more of specific alkyl (meth)acrylates, and at least one component selected from component Al to A5: a specific aldehyde compound, a specific acetal compound, a specific ketal compound, a specific carboxylic acid compound, or a specific alcohol, **in** which a contained amount of the ester compound (I) is 95% to 99.99% by mass with respect to the total mass of the ester-compound-containing composition.

EP 4 613 785 A1

## Description

### TECHNICAL FIELD

The present invention relates to an ester-compound-containing composition and a method for producing the same, a polymerizable composition, and a (meth)acrylic polymer and a method for producing the same.

Priority is claimed on Japanese Patent Application No. 2022-174445, Japanese Patent Application No. 2022-174450, and Japanese Patent Application No. 2022-174455, filed October 31, 2022, the contents of which are incorporated herein by reference.

### BACKGROUND ART

A (meth)acrylic polymer obtained from a (meth)acrylic acid ester is used in various fields such as a coating material, an adhesive, a resin reforming agent, artificial marble, and paper latex.

As a method for producing the (meth)acrylic acid ester, for example, a method in which a target (meth)acrylic acid alkyl ester is produced by an ester exchange reaction between a (meth)acrylic acid alkyl ester and an alkyl alcohol as raw materials in the presence of $Ti(OR)_4$ (R is an alkyl group) as a catalyst is known (for example, Patent Document 1).

### Citation List

#### Patent Document

Patent Document 1: Japanese Unexamined Patent Application, First Publication No. 2009-274986

### SUMMARY OF INVENTION

#### Technical Problem

When an ester-compound-containing composition containing a (meth)acrylic acid ester is stored for a certain period after production, quality of the ester-compound-containing composition may deteriorate.

An object of the present invention is to provide an ester-compound-containing composition and a method for producing the same, that can be produced efficiently and has excellent storage stability, a polymerizable composition using the ester-compound-containing composition, and a (meth)acrylic polymer and a method for producing the same.

#### Solution to Problem

As a result of intensive studies, the present inventors found that a decrease in purity concentration due to the generation of impurities such as a dimer of (meth)acrylic acid ester occurs during storage of the ester-compound-containing composition. The impurities such as a dimer of (meth)acrylic acid ester may cause a decrease in the physical properties of a (meth)acrylic polymer.

The present inventors conducted further studies, and found that, when the ester-compound-containing composition contains a specific component, the generation of impurities such as a dimer of (meth)acrylic acid ester is suppressed, and storage stability is improved, thereby completing the present invention.

That is, the present invention includes the following aspects.

[1] An ester-compound-containing composition, comprising:

one or more compounds selected from the group consisting of a compound represented by Formula (A1), a compound represented by Formula (A2), a compound represented by Formula (A3), a compound represented by Formula (A4), and a compound represented by Formula (A5); and
an ester compound (I) represented by Formula (1),
wherein a contained amount of the ester compound (I) is 95% to 99.99% by mass with respect to a total mass of the ester-compound-containing composition.

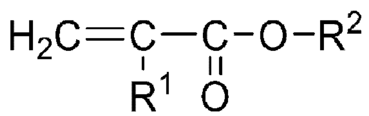

･･･(1)

(in Formula (1), $R^1$ is a hydrogen atom or a methyl group, and $R^2$ is a monovalent hydrocarbon group having 4 carbon atoms)

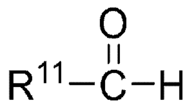 ···(A1)

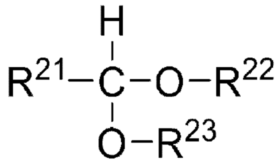 ···(A2)

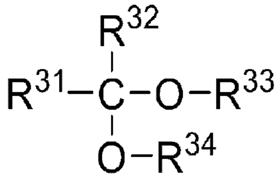 ···(A3)

(in Formulae (A1) to (A3), $R^{11}$ and $R^{21}$ are each independently a linear or branched alkyl group having 1 to 20 carbon atoms, and $R^{22}$ and $R^{23}$, $R^{31}$ and $R^{32}$, and $R^{33}$ and $R^{34}$ may be each independently a linear or branched alkyl group having 1 to 20 carbon atoms, or may be a divalent group having 1 to 20 carbon atoms, which may have an ether bond in combination with each of the groups)

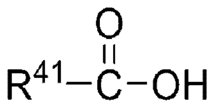 ···(A4)

(in Formula (A4), $R^{41}$ is a linear or branched alkyl group having 1 to 20 carbon atoms)

$R^{51}$-OH ▪ ▪ ▪ (A5)

(in Formula (A5), $R^{51}$ is a linear or branched alkyl group having 5 carbon atoms)

[2] The ester-compound-containing composition according to [1],
wherein the ester compound (I) includes one or more selected from the group consisting of butyl (meth)acrylate and isobutyl (meth)acrylate.
[3] The ester-compound-containing composition according to [1] or [2],
wherein the compounds represented by Formulae (A1) to (A5) are each at least one of butyraldehyde, 1,1-dibutoxybutane, 2,2-dibutoxypropane, 2,4-dimethyl-1,3-dioxane, 2,2,4-trimethyl-1,3-dioxane, 2-ethyl-4-methyl-1,3-dioxane, 1,3,5-trimethyl-2,4,6-trioxane, acetic acid, butyric acid, 1-pentyl alcohol, 2-methyl-1-butanol, or 3-methyl-1-butanol.
[4] The ester-compound-containing composition according to any one of [1] to [3],
wherein a total contained amount of the compounds represented by Formulae (A1) to (A5) is 1 ppm by mass or more and 1500 ppm by mass or less.
[5] The ester-compound-containing composition according to any one of [1] to [4], further comprising:
a component B: a polymerization inhibitor.
[6] The ester-compound-containing composition according to [5],
wherein component B is at least one polymerization inhibitor selected from the group consisting of a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.
[7] The ester-compound-containing composition according to [5],
wherein component B is at least one polymerization inhibitor selected from the group consisting of a phenol-based compound, an N-oxyl-based compound, an amine-based compound, a phosphorus-containing compound, and a sulfur-containing compound.

[8] The ester-compound-containing composition according to [5], wherein component B is at least one polymerization inhibitor selected from the group consisting of hydroquinone, 4-methoxyphenol, 2,4-dimethyl-6-t-butylphenol, and 2,6-di-t-butyl-4-methylphenol.
[9] The ester-compound-containing composition according to any one of [1] to [8], wherein the contained amount of the ester compound (I) is 99% to 99.99% by mass.
[10] The ester-compound-containing composition according to any one of [1] to [9], wherein the ester-compound-containing composition is stored for 1 day or longer.
[11] A method for producing an ester-compound-containing composition which contains an ester compound (I) represented by Formula (1), the production method comprising:

performing an ester exchange reaction between methyl (meth)acrylate and a monoalcohol (II) having 4 carbon atoms in the presence of one or more compounds selected from the group consisting of a compound represented by Formula (A1), a compound represented by Formula (A2), a compound represented by Formula (A3), a compound represented by Formula (A4), and a compound represented by Formula (A5).

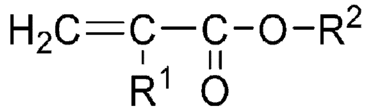 ··· (1)

(in Formula (1), $R^1$ is a hydrogen atom or a methyl group, and $R^2$ is a monovalent hydrocarbon group having 4 carbon atoms)

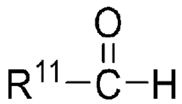 ··· (A1)

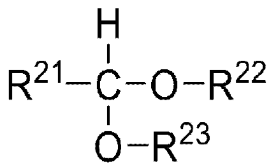 ··· (A2)

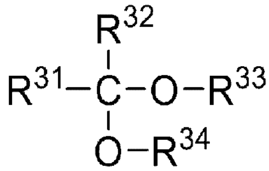 ··· (A3)

(in Formulae (A1) to (A3), $R^{11}$ and $R^{21}$ are each independently a linear or branched alkyl group having 1 to 20 carbon atoms, and $R^{22}$ and $R^{23}$, $R^{31}$ and $R^{32}$, and $R^{33}$ and $R^{34}$ may be each independently a linear or branched alkyl group having 1 to 20 carbon atoms, or may be a divalent group having 1 to 20 carbon atoms, which may have an ether bond in combination with each of the groups)

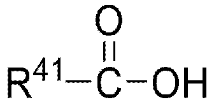 ··· (A4)

(in Formula (A4), $R^{41}$ is a linear or branched alkyl group having 1 to 20 carbon atoms)

$R^{51}$-OH ▪ ▪ ▪ (A5)

(in Formula (A5), $R^{51}$ is a linear or branched alkyl group having 5 carbon atoms)

[12] The method for producing an ester-compound-containing composition according to [11], wherein the monoalcohol (II) is at least one of n-butanol or isobutanol.

[13] The method for producing an ester-compound-containing composition according to [11] or [12],
wherein the compounds represented by Formulae (A1) to (A5) include at least one of butyraldehyde, 1,1-dibutoxybutane, 2,2-dibutoxypropane, 2,4-dimethyl-1,3-dioxane, 2,2,4-trimethyl-1,3-dioxane, 2-ethyl-4-methyl-1,3-dioxane, 1,3,5-trimethyl-2,4,6-trioxane, acetic acid, butyric acid, 1-pentyl alcohol, 2-methyl-1-butanol, or 3-methyl-1-butanol.
[14] A polymerizable composition for producing a (meth)acrylic polymer, comprising:
the ester-compound-containing composition according to any one of [1] to [10].
[15] The polymerizable composition according to [14], further comprising:
a monomer copolymerizable with the ester compound (I).
[16] A (meth)acrylic polymer obtained by polymerizing the polymerizable composition according to [14].
[17] A (meth)acrylic polymer obtained by polymerizing the polymerizable composition according to [15].
[18] A method for producing a (meth)acrylic polymer, comprising:
polymerizing the polymerizable composition according to [14].
[19] A method for producing a (meth)acrylic polymer, comprising:
polymerizing the polymerizable composition according to [15].

Advantageous Effects of Invention

According to the present invention, it is possible to provide an ester-compound-containing composition and a method for producing the same, that can be produced efficiently and has excellent storage stability, a polymerizable composition using the ester-compound-containing composition, and a (meth)acrylic polymer and a method for producing the same.

DESCRIPTION OF EMBODIMENTS

The present invention will be described in detail below. The following embodiments are merely examples explaining the present invention, but the present invention is not intended to be limited only to these embodiments. The present invention can be carried out in various aspects as long as they maintain the gist of the invention.
Definitions of the following terms in the present specification and claims are as follows.
"Monomer" means a compound having a polymerizable carbon-carbon double bond.
"(Meth)acrylate" is selected from "acrylate" and "methacrylate".
"(Meth)acrylic acid" is selected from "acrylic acid" and "methacrylic acid".
In the present specification, a numerical value range represented using "to" means a range including the numerical values listed before and after "to" as the lower limit value and the upper limit value. For example, "2 to 20" means 2 or more and 20 or less.

[Ester-compound-containing composition]

An ester-compound-containing composition according to an embodiment contains an ester compound (I) described later and a component A described later.
The ester-compound-containing composition according to the embodiment may contain, as necessary, a B component: a polymerization inhibitor, in addition to the ester compound (I) and component A. **In** addition, the ester-compound-containing composition according to the embodiment may further contain at least one of a compound other than the ester compound (I), component A, and component B, or water, as long as the effect of the present invention is not impaired.

(Ester compound (I))

The ester compound (I) is a compound represented by Formula (1).

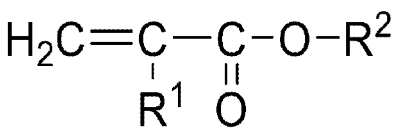 ···(1)

In Formula (1), $R^1$ is a hydrogen atom or a methyl group, and $R^2$ is a monovalent hydrocarbon group having 4 carbon atoms.
The hydrocarbon group of $R^2$ may be a saturated hydrocarbon group or an unsaturated hydrocarbon group.

The hydrocarbon group of $R^2$ may be linear or branched, or may have a ring. When the hydrocarbon group of $R^2$ has a ring, the ring may be an aliphatic ring or an aromatic ring.

Examples of the hydrocarbon group of $R^2$ include an n-butyl group, an isobutyl group, an s-butyl group, a t-butyl group, a crotyl group, a 3-butenyl group, and a cyclopropylmethyl group.

From the viewpoint that the ester compound (I) is relatively easy to obtain and is relatively easy to handle in terms of physical properties, $R^2$ is preferably an n-butyl group, an isobutyl group, an s-butyl group, a t-butyl group, or a crotyl group; more preferably an n-butyl group, an isobutyl group, an s-butyl group, or a t-butyl group; particularly preferably an n-butyl group or an isobutyl group; and most preferably an n-butyl group.

Examples of the ester compound (I) include butyl (meth)acrylate, isobutyl (meth)acrylate, s-butyl (meth)acrylate, t-butyl (meth)acrylate, crotyl (meth)acrylate, 3-butenyl (meth)acrylate, and cyclopropylmethyl (meth)acrylate.

From the viewpoint of relatively easy acquisition and relatively easy handling in terms of physical properties, as the ester compound (I), butyl (meth)acrylate, isobutyl (meth)acrylate, s-butyl (meth)acrylate, t-butyl (meth)acrylate, or crotyl (meth)acrylate is preferable; butyl (meth)acrylate, isobutyl (meth)acrylate, s-butyl (meth)acrylate, or t-butyl (meth) acrylate is more preferable; butyl (meth)acrylate or isobutyl (meth)acrylate is particularly preferable; and butyl (meth) acrylate is most preferable.

The ester compound (I) may be used alone or in combination of two or more kinds thereof.

(Component A)

Component A is one or more compounds selected from the group consisting of a compound represented by Formula (A1), a compound represented by Formula (A2), a compound represented by Formula (A3), a compound represented by Formula (A4), and a compound represented by Formula (A5).

Hereinafter, the compound represented by Formula (A1) will also be referred to as a compound (A1), and the compounds represented by other formulae will be similarly referred to.

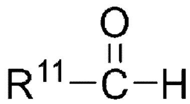

··· (A1)

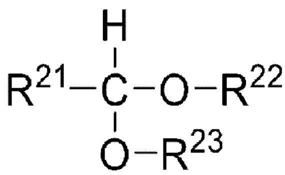

··· (A2)

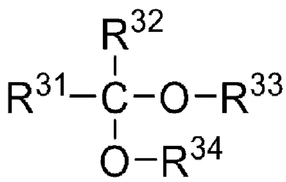

··· (A3)

In Formulae (A1) to (A3), $R^{11}$ and $R^{21}$ are each independently a linear or branched alkyl group having 1 to 20 carbon atoms. $R^{22}$ and $R^{23}$, $R^{31}$ and $R^{32}$, and $R^{33}$ and $R^{34}$ may be each independently a linear or branched alkyl group having 1 to 20 carbon atoms, or may be a divalent group having 1 to 20 carbon atoms, which may have an ether bond in combination with each of the groups.

Hereinafter, the compounds represented by Formulae (A1) to (A3) are also referred to as "component A1", "component A2", and "component A3".

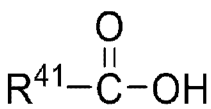

··· (A4)

In Formula (A4), $R^{41}$ is a linear or branched alkyl group having 1 to 20 carbon atoms.

Hereinafter, the compound represented by Formula (A4) is also referred to as "component A4".

$R^{51}$-OH ▪ ▪ ▪ (A5)

In Formula (A5), $R^{51}$ is a linear or branched alkyl group having 5 carbon atoms.

Hereinafter, the compound represented by Formula (A5) is also referred to as "component A5".

When the ester-compound-containing composition contains component A, a dimerization reaction of the ester compound (I) is suppressed during storage, and thus an ester-compound-containing composition having excellent storage stability is obtained. The reason for this is not clear, but is presumed as follows.

The dimerization reaction of the ester compound (I) occurs by generating a radical with ultraviolet rays such as sunlight and reacting with the ester compound (I). Since components A1 to A3 have a C-H bond which is easily homolyzed, the radical can be trapped. Therefore, components A1 to A3 can suppress the dimerization reaction of the ester compound (I), and can further suppress oxidation reaction of the ester compound (I).

In addition, the dimerization reaction of the ester compound (I) also proceeds by anionic polymerization under basic conditions. Since the carboxylic acid compound has weak acidity and can trap an anion which causes the anionic polymerization, component A4 can suppress the dimerization reaction of the ester compound (I) by anionic polymerization.

In addition, since the alcohol has weak acidity and can trap an anion which causes the anionic polymerization, component A5 can suppress the dimerization reaction of the ester compound (I) by anionic polymerization.

Furthermore, during the storage of the ester-compound-containing composition, for example, a radical derived from ultraviolet rays, such as a hydroxyl radical generated by absorption of ultraviolet rays derived from sunlight by oxygen molecules, is generated. Such a radical can cause generation of an oxidative product of the ester compound (I). The hydroxyl radical reacts with the alcohol as component A5 to be converted into an alkoxy radical having lower reactivity, and thus the generation of the oxidative product of the ester compound (I) is suppressed. In this case, when the alcohol and the ester compound (I) have few similarities in structure, a proportion of the hydroxyl radical reacting with the alcohol and the ester compound (I) can be biased toward the alcohol.

The alkyl group of $R^{11}$ in the compound (A1) may be linear or branched.

The number of carbon atoms in the alkyl group of $R^{11}$ is preferably 1 to 10 and more preferably 1 to 5.

Examples of the alkyl group of $R^{11}$ include a methyl group, an ethyl group, an n-propyl group, an isopropyl group, an n-butyl group, an isobutyl group, an s-butyl group, and a t-butyl group. Among these, an n-propyl group, an isopropyl group, or an n-butyl group is preferable, and an n-propyl group or an isopropyl group is more preferable.

Examples of the compound (A1) include acetaldehyde, propionaldehyde, butyraldehyde, isobutyraldehyde, and valeraldehyde. The compound (A1) may be used alone or in combination of two or more kinds thereof.

From the viewpoint that the storage stability of the ester-compound-containing composition is more excellent, the compound (A1) preferably includes one or more selected from butyraldehyde and isobutyraldehyde, and more preferably includes butyraldehyde.

The alkyl group of $R^{21}$ to $R^{23}$ in the compound (A2) may be linear or branched.

The number of carbon atoms in the alkyl group of $R^{21}$ is preferably 1 to 10 and more preferably 1 to 5.

Examples of the alkyl group of $R^{21}$ include a methyl group, an ethyl group, an n-propyl group, an isopropyl group, an n-butyl group, an isobutyl group, an s-butyl group, and a t-butyl group. Among these, a methyl group, an ethyl group, an n-propyl group, or an isopropyl group is preferable, a methyl group, an n-propyl group, or an isopropyl group is more preferable, and a methyl group or an n-propyl group is most preferable.

The number of carbon atoms in each alkyl group of $R^{22}$ and $R^{23}$ is preferably 1 to 10 and more preferably 1 to 5.

Examples of the alkyl group of $R^{22}$ and $R^{23}$ include a methyl group, an ethyl group, an n-propyl group, an isopropyl group, an n-butyl group, an isobutyl group, an s-butyl group, and a t-butyl group. Among these, an n-butyl group or an isobutyl group is preferable, and an n-butyl group is more preferable.

Examples of the divalent group having 1 to 20 carbon atoms, which may have an ether bond in which $R^{22}$ and $R^{23}$ are linked to each other, include a 1,3-propylene group, a 1,3-butylene group, a 1,4-butylene group, and $-CH(-CH_3)-O-CH(-CH_3)-$. Among these, a 1,3-butylene group or $-CH(-CH_3)-O-CH(-CH_3)-$ is preferable.

Examples of the compound (A2) include 1,1-diethoxyethane, 1,1-dibutoxyethane, 1,1-diisobutoxyethane, 1,1-diethoxybutane, 1,1-dibutoxybutane, 1,1-diethoxyisobutane, 1,1-diisobutoxyisobutane, 2-methyl-1,3-dioxane, 2,4-dimethyl-1,3-dioxane, 2,5-dimethyl-1,3-dioxane, 2-ethyl-4-methyl-1,3-dioxane, and 1,3,5-trimethyl-2,4,6-trioxane. The compound (A2) may be used alone or in combination of two or more kinds thereof.

From the viewpoint that the storage stability of the ester-compound-containing composition is more excellent, the compound (A2) preferably includes one or more selected from 1,1-diethoxybutane, 1,1-dibutoxybutane, 1,1-diisobutoxyisobutane, 2,4-dimethyl-1,3-dioxane, 2-ethyl-4-methyl-1,3-dioxane, and 1,3,5-trimethyl-2,4,6-trioxane; and more preferably includes 1,1-dibutoxybutane, 2,4-dimethyl-1,3-dioxane, or 1,3,5-trimethyl-2,4,6-trioxane.

The alkyl group of $R^{31}$ to $R^{34}$ in the compound (A3) may be linear or branched.

The number of carbon atoms in each alkyl group of $R^{31}$ and $R^{32}$ is preferably 1 to 10 and more preferably 1 to 5.

Examples of the alkyl group of $R^{31}$ and $R^{32}$ include a methyl group, an ethyl group, an n-propyl group, an isopropyl

group, an n-butyl group, an isobutyl group, an s-butyl group, and a t-butyl group. Among these, a methyl group, an ethyl group, an n-propyl group, or an isopropyl group is preferable, and a methyl group or an ethyl group is more preferable.

Examples of the divalent group having 1 to 20 carbon atoms, which may have an ether bond in which $R^{31}$ and $R^{32}$ are linked to each other, include a 1,4-butylene group, a 1,5-pentylene group, and a 1,6-hexylene group. Among these, a 1,5-pentylene group is preferable.

The number of carbon atoms in each alkyl group of $R^{33}$ and $R^{34}$ is preferably 1 to 10 and more preferably 1 to 5.

Examples of the alkyl group of $R^{33}$ and $R^{34}$ include a methyl group, an ethyl group, an n-propyl group, an isopropyl group, an n-butyl group, an isobutyl group, an s-butyl group, and a t-butyl group. Among these, an n-butyl group or an isobutyl group is preferable, and an n-butyl group is more preferable.

Examples of the divalent group having 1 to 20 carbon atoms, which may have an ether bond in which $R^{33}$ and $R^{34}$ are linked to each other, include a 1,3-propylene group, a 1,3-butylene group, a 1,4-butylene group, and $-CH(-CH_3)-O-CH(-CH_3)-$. Among these, a 1,3-butylene group or $-CH(-CH_3)-O-CH(-CH_3)-$ is preferable.

Examples of the compound (A3) include 2,2-dimethoxypropane, 2,2-diethoxypropane, 2,2-dibutoxypropane, 2,2-diisobutoxypropane, 2,2-dimethyl-1,3-dioxane, and 2,2,4-trimethyl-1,3-dioxane. The compound (A3) may be used alone or in combination of two or more kinds thereof.

From the viewpoint that the storage stability of the ester-compound-containing composition is more excellent, the compound (A3) preferably includes one or more selected from 2,2-diethoxypropane, 2,2-dibutoxypropane, 2,2-diisobutoxypropane, and 2,2,4-trimethyl-1,3-dioxane; and more preferably includes 2,2-dibutoxypropane or 2,2,4-trimethyl-1,3-dioxane.

The alkyl group of $R^{41}$ in the compound (A4) may be linear or branched.

The number of carbon atoms in the alkyl group of $R^{41}$ is preferably 1 to 20, more preferably 1 to 10, particularly preferably 1 to 7, and most preferably 1 to 5.

Examples of the alkyl group of $R^{41}$ include a methyl group, an ethyl group, an n-propyl group, an isopropyl group, an n-butyl group, an isobutyl group, an s-butyl group, a t-butyl group, and an n-pentyl group. Among these, a methyl group, an ethyl group, an n-propyl group, or an isopropyl group is preferable, and a methyl group or an n-propyl group is more preferable.

Examples of the compound (A4) include acetic acid, propionic acid, butyric acid, isobutyric acid, valeric acid, lauric acid, tridecylic acid, palmitic acid, and stearic acid. The compound (A4) may be used alone or in combination of two or more kinds thereof.

From the viewpoint that the storage stability of the ester-compound-containing composition is more excellent, the compound (A4) preferably includes one or more selected from the group consisting of acetic acid, propionic acid, butyric acid, and isobutyric acid, and more preferably includes at least one of acetic acid or butyric acid.

The alkyl group of $R^{51}$ in the compound (A5) may be linear or branched.

Examples of the alkyl group of $R^{51}$ include an n-pentyl group, a 2-pentyl group, a 3-pentyl group, a 2-methyl-1-butyl group, a 3-methyl-1-butyl group, a 2-methyl-2-butyl group, a 3-methyl-2-butyl group, and a neopentyl group. Among these, an n-pentyl group, a 2-methyl-1-butyl group, or a 3-methyl-1-butyl group is more preferable.

Examples of the compound (A5) include 1-pentyl alcohol, 2-pentyl alcohol, 3-pentyl alcohol, 2-methyl-1-butanol, 3-methyl-1-butanol, 2-methyl-2-butanol, 3-methyl-2-butanol, and neopentyl alcohol. The compound (A5) may be used alone or in combination of two or more kinds thereof.

From the viewpoint that the storage stability of the ester-compound-containing composition is more excellent, the compound (A5) is preferably one or more selected from the group consisting of 1-pentyl alcohol, 2-pentyl alcohol, 3-pentyl alcohol, 2-methyl-1-butanol, 3-methyl-1-butanol, 2-methyl-2-butanol, 3-methyl-2-butanol, and neopentyl alcohol; and more preferably one or more selected from the group consisting of 1-pentyl alcohol, 2-methyl-1-butanol, and 3-methyl-1-butanol.

From the viewpoint that the storage stability of the ester-compound-containing composition is more excellent, it is particularly preferable to contain, as component A, at least one of butyraldehyde, 1,1-dibutoxybutane, 2,2-dibutoxypropane, 2,4-dimethyl-1,3-dioxane, 2,2,4-trimethyl-1,3-dioxane, 2-ethyl-4-methyl-1,3-dioxane, 1,3,5-trimethyl-2,4,6-trioxane, acetic acid, butyric acid, 1-pentyl alcohol, 2-methyl-1-butanol, or 3-methyl-1-butanol.

(Component B)

Component B is a polymerization inhibitor.

When the ester-compound-containing composition contains component B, the progress of the polymerization reaction of the ester compound (I) by radical polymerization mechanism during storage can be suppressed, and a decrease in concentration of the ester compound (I) is easily suppressed. In addition, during storage, oxygen molecules in the ester-compound-containing composition absorb ultraviolet rays derived from sunlight, thereby generating a hydroxyl radical. However, the polymerization inhibitor can trap the hydroxyl radical. Therefore, when the ester-compound-containing composition contains component B, the amount of the hydroxyl radical can be reduced by component B

even when the hydroxyl radical is generated. Accordingly, the progress of dimerization of the ester compound (I) and the generation of the oxidative product can be suppressed more efficiently.

The "polymerization inhibitor" in the present invention means a compound having a function of suppressing the polymerization reaction of the ester compound (I).

Examples of component B include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

Examples of the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the nitrobenzene-based compound include nitrobenzene, o-dinitrobenzene, m-dinitrobenzene, p-dinitrobenzene, 2,4-dinitrobenzene, dinitrodurene, and 2,2-diphenyl-1-picrylhydrazine.

Examples of the N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethyl-piperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-t-butyl nitroxide, and di-t-amyl nitroxide.

Examples of the amine-based compound include N,N-diphenylamine, alkylated diphenylamine, 4,4'-dicamyldiphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, triphenylphosphite, tris(nonylphenyl)phosphite, 4,4'-isopropylidenediphenol alkylphosphite, tris(2,4-di-t-butylphenyl)phosphite, tris(biphenyl)phosphite, distearyl pentaerythritol diphosphite, di(2,4-di-t-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-t-butylphenol)

diphosphite, hexa(tridecyl)-1,1,3-tris(2-methyl-4-hydroxy-5-t-butylphenyl)butanetriphosphite, 3,5-dit-butyl-4-hydroxybenzyl phosphate diethyl ester, sodium-bis(4-t-butylphenyl)phosphate, sodium-2,2'-methylene-bis(4,6-di-t-butylphenyl)phosphate, and 1,3-bis(diphenoxyphosphoryloxy)benzene.

Examples of the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the iron-containing compound include iron (III) chloride.

Examples of the copper-containing compound include copper dimethyldithiocarbamate, copper diethylthiocarbamate, copper dibutylthiocarbamate, copper salicylate, copper acetate, copper thiocyanate, copper nitrate, copper chloride, copper carbonate, copper hydroxide, copper acrylate, and copper methacrylate.

Examples of the manganese-containing compound include manganese dialkyldithiocarbamate, manganese diphenyldithiocarbamate, manganese formate, manganese acetate, manganese octanoate, manganese naphthenate, manganese permanganate, and manganese ethylenediaminetetraacetate. Alkyl groups of the manganese dialkyldithiocarbamate are any one of a methyl group, an ethyl group, a propyl group, or a butyl group, and may be the same or different from each other.

From the viewpoint of easily exhibiting the effect of further improving the storage stability of the ester-compound-containing composition, as component B, one or more polymerization inhibitors selected from the group consisting of a phenol-based compound, an N-oxyl-based compound, an amine-based compound, a phosphorus-containing compound, and a sulfur-containing compound are preferable; one or more polymerization inhibitors selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine are more preferable; and one or more polymerization inhibitors selected from the group consisting of hydroquinone, 4-methoxyphenol, 2,4-dimethyl-6-t-butylphenol, and 2,6-di-t-butyl-4-methylphenol are particularly preferable.

Component B may be used alone or in combination of two or more kinds thereof.

When the ester-compound-containing composition contains a compound corresponding to both component A1 and component B, the compound is regarded as component A1. When the ester-compound-containing composition contains component A1 and component B, this means that the ester-compound-containing composition further contains component B different from the compound.

When the ester-compound-containing composition contains two or more kinds of compounds corresponding to both component A1 and component B, a compound having the highest molar concentration in the ester-compound-containing composition is regarded as component A1, and the other compounds are regarded as component B.

Components A2 to A5 are also regarded in the same manner as component A1.

(Component C)

Component C is a compound other than the ester compound (I), component A, and component B.

Examples of component C include additives such as a release agent, a lubricant, a plasticizer, an antioxidant, an antistatic agent, a light stabilizer, an ultraviolet absorber, a flame retardant, a flame retardant aid, a filler, a pigment, a dye, a silane coupling agent, a leveling agent, an antifoaming agent, and a fluorescent agent.

The ester-compound-containing composition may contain unreacted raw materials in the production of the ester-compound-containing composition, such as methyl (meth)acrylate, alcohol, and (meth)acrylic acid.

The ester-compound-containing composition may contain impurities generated during the production of the ester-compound-containing composition, such as diacetyl, but from the viewpoint of suppressing coloration of the ester-compound-containing composition, the concentration of the diacetyl is preferably 5 ppm by mass or less, more preferably 2 ppm by mass or less, still more preferably 1 ppm by mass or less, and particularly preferably 0.1 ppm by mass or less.

The ester-compound-containing composition may contain a (meth)acrylic acid ester other than the ester compound (I).

(Formulation)

The contained amount of the ester compound (I) in the ester-compound-containing composition is 95% to 99.99% by mass with respect to the total mass of the ester-compound-containing composition. When the concentration of the ester compound (I) is 95% by mass or more, the amount of impurities when the (meth)acrylic polymer is produced by polymerization of the ester-compound-containing composition can be reduced, and thus it is possible to prevent physical properties of the polymer from being adversely affected. In addition, when the concentration of the ester compound (I) is 99.99% by mass or less, a purification cost can be reduced. The concentration of the ester compound (I) is preferably 95% by mass or more, more preferably 96% by mass or more, still more preferably 98% by mass or more, particularly preferably 99% by mass or more, and most preferably 99.5% by mass or more.

A contained amount of component A in the ester-compound-containing composition is not particularly limited, but is preferably 1 to 10,000 ppm by mass with respect to the total mass of the ester-compound-containing composition. When the contained amount thereof is 1 ppm by mass or more, an effect of suppressing the generation of the dimer of the ester compound (I) can be sufficiently obtained. In addition, when the contained amount thereof is 10,000 ppm by mass or less, the amount of impurities when the (meth)acrylic polymer is produced by polymerization of the ester-compound-containing composition according to the embodiment of the present invention can be reduced, and thus it is possible to prevent physical properties of the polymer from being adversely affected. The contained amount of component A is more preferably 3 ppm by mass or more, still more preferably 5 ppm by mass or more, and particularly preferably 10 ppm by mass or more. The contained amount of component A is more preferably 7,500 ppm by mass or less, still more preferably 5,000 ppm by mass or less, even more preferably 2,500 ppm by mass or less, even still more preferably 1,500 ppm by mass or less, particularly preferably 1,000 ppm by mass or less, and most preferably 500 ppm by mass or less.

The lower limit and the upper limit of the preferred contained amount of component A can be optionally combined, and for example, the contained amount thereof is preferably 1 to 1,500 ppm by mass and more preferably 10 to 1,000 ppm by mass.

The contained amount of component B in the ester-compound-containing composition is not particularly limited, but is preferably 1 to 1,000 ppm by mass with respect to the total mass of the ester-compound-containing composition. When the contained amount thereof is 1 ppm by mass or more, an effect of suppressing the generation of the dimer of the ester compound (I) can be sufficiently obtained. In addition, when the contained amount thereof is 1,000 ppm by mass or less, the amount of impurities when the (meth)acrylic polymer is produced by polymerization of the ester-compound-containing composition according to the embodiment of the present invention can be reduced, and thus it is possible to prevent physical properties of the polymer from being adversely affected. The contained amount of component B is more preferably 3 ppm by mass or more, still more preferably 5 ppm by mass or more, and particularly preferably 10 ppm by mass or more. The contained amount of component B is more preferably 750 ppm by mass or less, still more preferably 500 ppm by mass or less, even more preferably 250 ppm by mass or less, particularly preferably 100 ppm by mass or less, and most preferably 50 ppm by mass or less.

The lower limit and the upper limit of the preferred contained amount of component B can be optionally combined, and for example, the contained amount thereof is preferably 1 to 750 ppm by mass, more preferably 3 to 500 ppm by mass, and still more preferably 5 to 100 ppm by mass.

A moisture content of the ester-compound-containing composition is preferably 5,000 ppm by mass or less, more preferably 4,000 ppm by mass or less, still more preferably 3,000 ppm by mass or less, particularly preferably 2,000 ppm by mass or less, and most preferably 1,000 ppm by mass or less with respect to the total mass of the ester-compound-containing composition. When the moisture content of the ester-compound-containing composition is the above-described upper limit value or less, the physical properties of the (meth)acrylic polymer obtained by polymerizing the ester-compound-containing composition can be maintained more satisfactorily.

The lower limit value of the moisture content of the ester-compound-containing composition is 0 ppm by mass.

(Analysis of ester-compound-containing composition)

Component A, component B, component C, and the water contained in the ester-compound-containing composition can be confirmed by, for example, GC-MS measurement.

In a GC-MS chart of the ester-compound-containing composition, when a peak is present at the same retention time as a sample of component A and an m/z value detected in a mass spectrum of the peak matches exact mass of component A, it can be determined that the ester-compound-containing composition contains component A. When a sample of component A cannot be obtained, when a pattern of the mass spectrum of the peak appearing in the GC-MS chart of the ester-compound-containing composition and a pattern of a mass spectrum of component A in mass spectrum database and match each other, it can be determined that the peak is the peak of component A and the ester-compound-containing composition contains component A. Examples of the mass spectrum database include NIST 20, NIST 17, NIST 14, and NIST 14s. In addition, when volatility is low and the detection cannot be carried out by the GC-MS measurement, the detection can be carried out by LC-MS. Component B, component C, and water can also be confirmed by the same method as that for component A.

The contained amount of the ester compound (I) can be calculated by performing GC-FID measurement of the ester-compound-containing composition, quantifying by an area percentage method, and correcting the quantified moisture content using a Karl Fischer moisture meter.

The concentration of component A can be quantified, for example, by performing GC measurement or GC-MS measurement of the ester-compound-containing composition and using an internal standard method or an absolute calibration curve method. When a sample of component A cannot be obtained and component A cannot be quantified by the internal standard method or the absolute calibration curve method, the concentration of component A can be calculated using the following expression by performing GC-FID measurement on any organic compound having a

known concentration under the same conditions as those of the ester-compound-containing composition.

$$\text{Contained amount of component A (ppm by mass)} = \frac{N}{N_A} \times \frac{S_A}{S} \times M$$

In the expression, N is the number of carbon atoms in one molecule of any organic compound, $N_A$ is the number of carbon atoms in one molecule of component A, $S_A$ is a peak area of component A, S is a peak area of the organic compound, and M is a contained amount (ppm by mass) of any organic component.
When the volatility is low and the quantification cannot be performed based on the GC area, the quantification can be performed using a chromatography method such as LC.
The contained amounts of component B and component C can also be calculated by the same method as that for component A.
The moisture content of the ester-compound-containing composition can be confirmed by the Karl Fischer method.

[Method for producing ester-compound-containing composition]

The ester-compound-containing composition as an example according to the embodiment of the present invention can be produced, for example, by performing an ester exchange reaction between methyl (meth)acrylate and a monoalcohol (II) having 4 carbon atoms in the presence of component A.
Among (meth)acrylic acid esters, the methyl (meth)acrylate tends to particularly easily undergo dimerization or oxidation reaction. However, by performing the ester exchange reaction in the presence of component A, the dimerization of methyl (meth)acrylate is suppressed, and as a result, the yield of the ester compound (I) is improved. In addition, by performing the ester exchange reaction in the presence of component A and component B, the yield of the ester compound (I) is further improved.
The ester-compound-containing composition containing the ester compound (1) can be produced by performing an ester exchange reaction between methyl (meth)acrylate and the monoalcohol (II) in the presence of component A.
More specifically, for example, in a reaction vessel, the methyl (meth)acrylate is subjected to an ester exchange with the monoalcohol (II) as represented by Formula (2) in the presence of a catalyst and component A. Here, $R^1$ and $R^2$ in Formula (2) are the same as $R^1$ and $R^2$ in Formula (1).

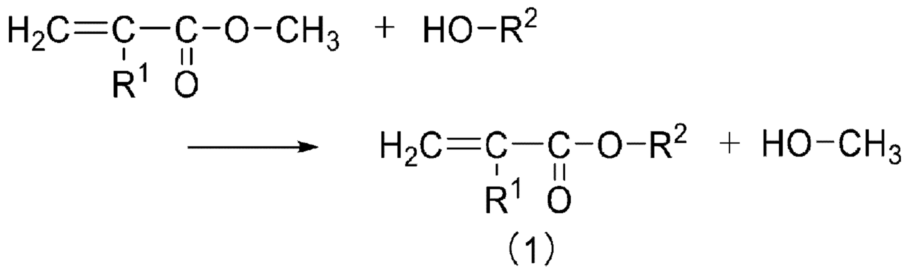

•••(2)

(1)

The monoalcohol (II) may be linear or branched.
Examples of the monoalcohol (II) include n-butanol, isobutanol, s-butanol, t-butanol, crotyl alcohol, and cyclopropyl methanol.
The monoalcohol (II) is preferably at least one of n-butanol or isobutanol.
The monoalcohol (II) may be used alone or in combination of two or more kinds thereof.
As the monoalcohol (II), an alcohol derived from a biomass may be used.
The alcohol derived from a biomass is not particularly limited, and for example, butanol obtained by an ABE method, which produces acetone, butanol, and ethanol by fermenting biomass using Clostridium bacteria, can be used.
The reaction vessel is preferably a reaction vessel including a distillation column. Since the ester exchange reaction is an equilibrium reaction, productivity is improved by separating a by-produced methanol by the distillation column. For example, it is preferable to perform the ester exchange reaction while separating methanol as a azeotropic mixture with the methyl (meth)acrylate to the outside of the system.
Examples of the reaction vessel include a reaction vessel including a distillation column provided on an upper part of a reaction container called a reaction kettle, and a distillation column in which a distillation can can be used as a reaction container. Examples of the distillation column include a packed column-type distillation column and a tray-type distillation column.

From the viewpoint of high separation ability and stable operation, a theoretical number of column plates of the distillation column is preferably 5 or more, and more preferably 7 or more.

A ratio of the amount of the methyl (meth)acrylate to be charged and the amount of the monoalcohol (II) to be charged can be appropriately determined. From the viewpoint of improving productivity, the ratio of the methyl (meth) acrylate to 1 mol of the monoalcohol (II) is preferably 0.1 to 10 mol, and more preferably 0.3 to 4 mol.

The catalyst to be used is not particularly limited, and examples thereof include hydroxides, carbonates, and bicarbonates of an alkali metal such as lithium, sodium, and potassium; oxides, hydroxides, and carbonates of an alkaline earth metal such as magnesium and calcium; alkali metal alkoxides such as lithium methoxide, sodium methoxide, sodium ethoxide, and potassium t-butoxide; alkali metal amides such as lithium amide, sodium amide, and potassium amide; titanium alkoxides such as tetramethyl titanate, tetraethyl titanate, tetrapropyl titanate, triisopropyl titanate, tetrabutyl titanate, and tetra(2-ethylhexyl) titanate; and tin-based compounds such as dibutyl tin oxide and dioctyl tin oxide. Among these, from the viewpoint of small amount of by-products of Michael addition reaction product during the ester exchange reaction and high catalytic activity, an alkoxide of titanium, dibutyltin oxide, or dioctyltin oxide is preferable. The catalyst may be used alone or in combination of two or more kinds thereof.

The catalyst can be supplied alone to the reaction vessel, or be supplied to the reaction vessel in a dissolved state in the same alcohol as the monoalcohol (II) as the raw material, or in a dissolved state in methyl (meth)acrylate as the raw material. Examples thereof include a method of directly dissolving the catalyst in the total amount of the monoalcohol (II) used in the reaction and supplying the catalyst to the reaction vessel, and a method of dissolving the catalyst in a part of the monoalcohol (II) used in the reaction and supplying the catalyst to the reaction vessel.

The amount of the catalyst used is preferably 0.001 to 1 mol% and more preferably 0.01 to 0.1 mol% with respect to 1 mol of the monoalcohol (II).

A solvent may be used for the ester exchange reaction. When a solvent is used, it is preferable to use a solvent which forms an azeotropic formulation with the by-produced methanol.

Examples of the solvent include n-pentane, n-hexane, n-heptane, n-octane, 2,3-dimethylbutane, 2,5-dimethylhexane, 2,2,4-trimethylpentane, cyclohexane, benzene, and toluene. Among these, n-hexane, n-heptane, or cyclohexane is preferable. The solvent may be used alone or in combination of two or more thereof.

A reaction temperature for the ester exchange reaction varies depending on the type of the monoalcohol (II) or the solvent, but is preferably 60°C to 150°C.

A reaction pressure for the ester exchange reaction is not particularly limited, and the reaction may be carried out under any pressure of reduced pressure, normal pressure, or elevated pressure.

The form of the ester exchange reaction is not particularly limited, and the ester exchange reaction can be performed by a generally used method, for example, a batch method, a continuous method, or the like.

After the ester exchange reaction, unreacted raw materials and by-products may be separated by purifying the reaction solution. For the purification, for example, a known method such as distillation, crystallization, extraction, and column chromatography can be used.

The method for producing an ester-compound-containing composition according to the present embodiment is not limited to the above-described method.

For example, other (meth)acrylic acid esters other than the methyl (meth)acrylate may be used as the raw material instead of the methyl (meth)acrylate.

An esterification reaction between the monoalcohol (II) and (meth)acrylic acid may be carried out to produce the ester compound (I).

In addition, component B may be blended into a solution containing the ester compound (I) after the ester exchange reaction and component A. In this case, a solution (B solution) containing the ester compound (I) and component B is prepared separately from solution (A solution) containing the ester compound (I) and component A, and the A solution and the B solution are mixed with each other to obtain the ester-compound-containing composition. In addition, the ester compound (I), the A solution, and the B solution may be mixed with each other to obtain the ester-compound-containing composition.

[Evaluation method for storage stability and thermal stability of ester-compound-containing composition]

The ester-compound-containing composition according to the embodiment of the present invention has high quality stability during storage. Examples of an evaluation method of the quality stability of the ester-compound-containing composition during storage include a method of actually storing the ester-compound-containing composition for a long period of time and confirming a generation amount of the dimer of the ester compound (I), the oxidative product of the ester compound (I), and a contained amount of the ester compound (I). In addition, from the viewpoint of ease of work, a method of heating the ester-compound-containing composition for a short time and confirming generation amounts of the dimer of the ester compound (I) and the oxidative product, and a contained amount of the ester compound (I) may be used. When heating for a short time, a heating temperature is preferably 50°C to 100°C and a heating time is preferably 1 to 24 hours. In the present invention, when the ester-compound-containing composition is stored at 25°C for 14 days or heated at 70°C for 7 to 20 hours, the quality stability of the ester-compound-containing composition during storage is evaluated based on the

generation amounts of the dimer of the ester compound (I) and the oxidative product, and the contained amount of the ester compound (I).

[Polymerizable composition]

The polymerizable composition as an example according to the embodiment of the present invention is a polymerizable composition for producing a (meth)acrylic polymer, and contains the above-described ester-compound-containing composition according to the embodiment of the present invention.
As an example of the embodiment, an ester-compound-containing composition stored for 1 day or more after production can be used as the polymerizable composition.
A storage time of the ester-compound-containing composition can be 1 day or more, 3 days or more, 7 days or more, or 14 days or more. In addition, the storage time of the ester-compound-containing composition may be 180 days or less, 150 days or less, 120 days or less, or 90 days or less. The "storage time" means an elapsed time from immediately after the production, which is not limited to a time during which the product is left to stand under a specific environment and also includes a time of transportation or the like.
A material of a storage container for the ester-compound-containing composition is not particularly limited, and for example, a metal container such as stainless steel, a resin container, or a glass container can be used. A transparent container or an opaque container can be used.
A temperature at the time of storing the ester-compound-containing composition is preferably -10°C or higher and more preferably 0°C or higher, and is preferably 60°C or lower and more preferably 50°C or lower. By setting the temperature to -10°C or higher, a load on a cooling device can be reduced, and by setting the temperature to 60°C or lower, it is easy to suppress the generation of the dimerized substance or the oxidative product of (meth)acrylic acid ester.
An oxygen concentration of a gas phase portion at the time of storing the ester-compound-containing composition is preferably 5% by volume or more and more preferably 7% by volume or more, and is preferably 30% by volume or less and more preferably 22% by volume or less. By setting the oxygen concentration of the gas phase portion to 5% by volume or more, it is easy to prevent the ester compound from being unintentionally polymerized by a polymerization prevention effect of oxygen, and by setting the oxygen concentration of the gas phase portion to 30% by volume or less, it is easy to suppress oxidation of the (meth)acrylic acid ester by oxygen and the generation of various impurities.
As an embodiment, the ester-compound-containing composition after storage may be used in the polymerizable composition without further blending a monomer, or a monomer (hereinafter, also referred to as "other monomer") copolymerizable with the ester compound (I) may be further blended with the ester-compound-containing composition after storage to obtain the polymerizable composition.
As an example of the embodiment, the ester-compound-containing composition after the production may be stored in a state in which the other monomer is blended, and used in the polymerizable composition. In this case, it is preferable that a polymerization initiator is not blended during the storage.
A contained amount of the ester compound (I) with respect to the total mass of monomers in the polymerizable composition is preferably 10% by mass or more and more preferably 20% by mass or more, and preferably 90% by mass or less and more preferably 80% by mass or less.
The above-described upper limit and lower limit of the contained amount of the ester compound (I) can be combined arbitrarily. For example, the contained amount of the ester compound (I) is preferably 10% to 90% by mass and more preferably 20% to 80% by mass.
Examples of the other monomer include methyl (meth)acrylate, unsaturated carboxylic acid, unsaturated carboxylic acid anhydride, maleimide, a hydroxy group-containing vinyl monomer, vinyl ester, a nitrogen-containing vinyl monomer, an epoxy group-containing monomer, an aromatic vinyl monomer, alkanediol di(meth)acrylate, polyoxyalkylene glycol di(meth)acrylate, and a vinyl monomer having two or more ethylenically unsaturated bonds in the molecule.
Examples of the unsaturated carboxylic acid include acrylic acid, methacrylic acid, maleic acid, and itaconic acid.
Examples of the unsaturated carboxylic acid anhydride include maleic acid anhydride and itaconic acid anhydride.
Examples of the maleimide include N-phenylmaleimide and N-cyclohexylmaleimide.
Examples of the hydroxy group-containing vinyl monomer include 2-hydroxyethyl acrylate, 2-hydroxyethyl methacrylate, and 2-hydroxypropyl methacrylate.
Examples of the vinyl ester include vinyl acetate and vinyl benzoate.
Examples of the nitrogen-containing vinyl monomer include methacrylamide and acrylonitrile.
Examples of the epoxy group-containing monomer include glycidyl acrylate and glycidyl methacrylate.
Examples of the aromatic vinyl monomer include styrene and $\alpha$-methylstyrene.
Examples of the alkanediol di(meth)acrylate include ethylene glycol di(meth)acrylate, 1,2-propylene glycol di(meth)acrylate, 1,3-butylene glycol di(meth)acrylate, and 1,6-hexanediol di(meth)acrylate.
Examples of the polyoxyalkylene glycol di(meth)acrylate include diethylene glycol di(meth)acrylate, dipropylene

glycol di(meth)acrylate, triethylene glycol (meth)acrylate, tetraethylene glycol di(meth)acrylate, polyethylene glycol di(meth)acrylate, and neopentyl glycol di(meth)acrylate.

Examples of the vinyl monomer having two or more ethylenically unsaturated bonds in the molecule include divinylbenzene.

As the other monomer, vinyl chloride, vinylidene chloride, derivatives thereof, an unsaturated polyester prepolymer obtained from at least one polycarboxylic acid including an ethylenically unsaturated polycarboxylic acid and at least one diol, and a vinyl ester prepolymer obtained by acrylic modification of a terminal of an epoxy group may be used.

The other monomer may be used alone or in combination of two or more kinds thereof.

When component A is a monomer copolymerizable with the ester compound (I), component A may be used as the monomer copolymerizable with the ester compound (I), or a monomer copolymerizable with the ester compound (I) may be used separately from component A.

A contained amount of the other monomer in the polymerizable composition is preferably 0 to 50 parts by mass with respect to 100 parts by mass of the ester compound (I). As a result, a (meth)acrylic polymer having high transparency can be obtained. The upper limit of the contained amount of the other monomer is more preferably 40 parts by mass or less and still more preferably 30 parts by mass or less with respect to 100 parts by mass of the ester compound (I). The lower limit of the contained amount of the other monomer is more preferably 0.01 parts by mass or more, still more preferably 0.1 parts by mass or more, and particularly preferably 1 part by mass or more with respect to 100 parts by mass of the ester compound (I).

The above-described upper limit and lower limit of the contained amount of the other monomer can be combined arbitrarily. For example, the contained amount of the other monomer is preferably 0 to 50 parts by mass, more preferably 0.01 to 40 parts by mass, still more preferably 0.1 to 30 parts by mass, and particularly preferably 1 to 30 parts by mass with respect to 100 parts by mass of the ester compound (I).

The polymerizable composition according to the present embodiment preferably contains a polymerization initiator.

Examples of the polymerization initiator include an azo compound, an organic peroxide, a persulfate compound, and a redox polymerization initiator.

The polymerization initiator may be used alone or in combination of two or more thereof.

Examples of the azo compound include 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate.

Examples of the organic peroxide include benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxy-isononanoate, di-t-butyl peroxide, di-t-hexyl peroxide, lauroyl peroxide, and dilauroyl peroxide.

Examples of the persulfate compound include potassium persulfate.

The amount of polymerization initiator added is not particularly limited, and for example, can be 0.005 to 5 parts by mass with respect to 100 parts by mass of the total mass of the monomers in the polymerizable composition.

The polymerizable composition according to the present embodiment may contain, as necessary, other additives such as a chain transfer agent, a release agent, a lubricant, a plasticizer, an antioxidant, an antistatic agent, a light stabilizer, an ultraviolet absorber, a flame retardant aid, a flame retardant assistant, a filler, a pigment, a dye, a silane coupling agent, a leveling agent, an antifoaming agent, and a fluorescent agent.

The other additives may be used alone or in combination of two or more thereof.

[Method for producing (meth)acrylic polymer]

A (meth)acrylic polymer can be produced by polymerizing the polymerizable composition as an example according to the present embodiment.

The polymerization method is not particularly limited, and examples thereof include a bulk polymerization method, a solution polymerization method, an emulsion polymerization method, and a suspension polymerization method. From the viewpoint of environmental load due to the use of the solvent or the like and viewpoint of transparency of the obtained (meth)acrylic polymer, a bulk polymerization method is preferable.

Specific methods of the bulk polymerization method are not particularly limited, and examples thereof include known casting polymerization methods such as a cell casting method and a continuous casting method.

The casting polymerization method is a method in which the (meth)acrylic polymer is obtained by casting and

polymerizing the polymerizable composition in a mold made of two inorganic glass plates or metal plates (for example, SUS plates) arranged facing each other at a predetermined interval with the periphery sealed with a gasket such as a soft resin tube.

The mold for the casting polymerization is not particularly limited, and a known mold can be used. Examples of a mold for cell casting include a mold in which two plate-shaped products such as an inorganic glass plate, a chromium-plated metal plate, and a stainless steel plate are arranged facing each other at a predetermined interval, and a gasket is disposed on edges of the plates to form a sealed space between the plate-shaped products and the gasket. Examples of the mold for continuous casting include a mold in which a sealed space is formed by opposing surfaces of a pair of endless belts running in the same direction at the same speed and gaskets running at the same speed as the endless belt on both sides of the endless belt.

A gap between cavities of the molds is appropriately adjusted to obtain a resin plate having a desired thickness, and is generally 1 to 30 mm.

A polymerization temperature is preferably 125°C to 210°C and more preferably 130°C to 180°C.

A polymerization time is preferably 0.5 to 24 hours.

A weight-average molecular weight (Mw) of the (meth)acrylic polymer is not particularly limited, and can be, for example, 100,000 to 1,000,000. As the Mw of the (meth)acrylic polymer becomes higher, solvent resistance and chemical resistance can be further improved.

The Mw of the (meth)acrylic polymer can be controlled by adjusting the polymerization temperature, the polymerization time, the amount of polymerization initiator added, and the like.

The (meth)acrylic polymer according to the embodiment of the present invention has excellent heat resistance and excellent meltability. For example, among (meth)acrylic polymers, a granular (meth)acrylic polymer having a large amount of resin which can be stored per unit volume and having a small energy cost during storage and transportation needs to be dissolved or melted at the time of use. However, the (meth)acrylic polymer according to the embodiment of the present invention is easily melted, has excellent kneading properties with other resins, and has excellent solubility in a monomer, a solvent, or the like.

The present invention will be specifically described below with reference to Examples, but the present invention is not limited by the following description. Unless otherwise specified, "%" and "ppm" in Examples and Comparative Examples mean "% by mass" and "ppm by mass".

A moisture concentration was calculated by the Karl Fischer method. A formulation of constituent components of the ester-compound-containing composition before storage was calculated from the amount of each raw material added. A dimer of the ester compound (I) and an oxidative product in the ester-compound-containing composition before and after storage were quantified by an absolute calibration curve method using GC-MS.

[GC-MS measurement]

Device: GC-MS measuring device (product name: QP-2010, manufactured by Shimadzu Corporation)

(GC conditions)

Column (product name: DB-FFAP, manufactured by Agilent)
length: 30 m, inner diameter: 0.25 mm, film thickness: 0.25 μm
Injection volume: 1.0 μL
Vaporization chamber temperature: 240°C
Column oven temperature: held at 60°C for 3 minutes, raised from 60°C to 240°C at 10 °C/min, and held at 240°C for 4 minutes
Carrier gas: helium
Injection mode: split (split ratio: 30)
Control mode: constant linear velocity (50.0 cm/sec)
Pressure: 116.3 KPa
Total flow rate: 61.3 mL/min
Purge flow rate: 3.0 mL/min
Column flow rate: 1.88 mL/min

(MS conditions)

Ionization method: electron ionization (EI)
Ion source temperature: 240°C
Interface temperature: 240°C
m/z detection range: 10 to 500
Detection time: 25 minutes

[Measurement of moisture content]

A moisture content was quantified by the Karl Fischer method using a trace automatic moisture content-measuring device (product name: CA-21, manufactured by Nittoseiko Analytech Co., Ltd.).

[Example 1]

0.020 g of 2-methyl-1-butanol as a component A was added to 9.919 g of butyl methacrylate (BMA, manufactured by Tokyo Chemical Industry Co., Ltd., moisture content: 120 ppm by mass) as an ester compound (I) to prepare an A solution.
In addition, 0.020 g of 2,4-dimethyl-6-t-butylphenol as a component B was added to 9.924 g of BMA (moisture content: 120 ppm by mass) as an ester compound (I) to prepare a B solution.
Next, 0.050 g of the A solution and 0.050 g of the B solution were added to 9.850 g of BMA (moisture content: 120 ppm by mass) as an ester compound (I) to prepare an ester-compound-containing composition.
The obtained ester-compound-containing composition was stored at 25°C for 14 days under white LED illumination, a contained amount (mg/L) of a BMA dimer and a contained amount (mg/L) of butyl pyruvate were determined by the GC-MS measurement using the absolute calibration curve method, and the amount of change (generated amount) before and after storage was calculated.

[Examples 2 to 37 and Comparative Examples 1 to 8]

An ester-compound-containing composition was prepared in the same manner as in Example 1, except that the formulations of the A solution, the B solution, a C solution (solution containing a component C), and the ester-compound-containing composition were changed as shown in Tables 1 to 3, and the generated amounts of the BMA dimer and the butyl pyruvate were obtained.

[Table 1]

| | A solution | | | | B solution | | | | C solution | | | | Ester-compound-containing composition | | | |
|---|---|---|---|---|---|---|---|---|---|---|---|---|---|---|---|---|
| | BMA [g] | Component A | | | BMA [g] | Component B | | | BMA [g] | Component C | | | | BMA [g] | A solution [g] | B solution [g] | C solution [g] |
| | | Compound name | Addition amount [g] | Contained amount [ppm by mass] | | Compound name | Addition amount [g] | Contained amount [ppm by mass] | | Compound name | Addition amount [g] | Contained amount [ppm by mass] | | | | |
| Example 1 | 9.919 | 2-Methyl-1-butanol | 0.020 | 2032 | 9.924 | DMTBP | 0.020 | 2041 | - | - | - | - | 9.850 | 0.050 | 0.050 | - |
| Example 2 | 9.919 | 2-Methyl-1-butanol | 0.020 | 2032 | 9.924 | DMTBP | 0.020 | 2041 | - | - | - | - | 9.435 | 0.503 | 0.050 | - |
| Example 3 | 9.919 | 2-Methyl-1-butanol | 0.020 | 2032 | 9.924 | DMTBP | 0.020 | 2041 | - | - | - | - | 4.933 | 5.001 | 0.050 | - |
| Example 4 | 10.000 | 2-Methyl-1-butanol | 1.001 | 90952 | 9.924 | DMTBP | 0.020 | 2041 | - | - | - | - | 8.849 | 1.100 | 0.050 | - |
| Example 5 | 9.919 | 2-Methyl-1-butanol | 0.020 | 2032 | 9.924 | DMTBP | 0.020 | 2041 | - | - | - | - | 9.473 | 0.050 | 0.501 | - |
| Example 6 | 9.919 | 2-Methyl-1-butanol | 0.020 | 2032 | 9.924 | DMTBP | 0.020 | 2041 | - | - | - | - | 4.930 | 0.050 | 5.000 | - |
| Example 7 | 9.919 | 2-Methyl-1-butanol | 0.020 | 2032 | - | - | - | - | - | - | - | - | 9.899 | 0.050 | - | - |
| Example 8 | 10.004 | 2-Methyl-1-butanol | 0.021 | 2055 | 10.00 4 | MEHQ | 0.020 | 2015 | - | - | - | - | 10.00 6 | 0.051 | 0.051 | - |
| Example 9 | 10.004 | 2-Methyl-1-butanol | 0.021 | 2055 | 10.00 1 | IPPDA | 0.020 | 1996 | - | - | - | - | 10.02 7 | 0.050 | 0.052 | - |
| Example 10 | 10.004 | 2-Methyl-1-butanol | 0.021 | 2055 | 10.00 2 | PTZ | 0.020 | 2016 | - | - | - | - | 10.00 7 | 0.050 | 0.051 | - |
| Example 11 | 10.004 | 2-Methyl-1-butanol | 0.021 | 2055 | 10.00 2 | HO-TEMPO | 0.020 | 1996 | - | - | - | - | 10.00 1 | 0.050 | 0.051 | - |
| Example 12 | 10.001 | 2-Methyl-1-butanol | 0.020 | 2016 | 10.00 1 | DMTBP | 0.020 | 2036 | - | - | - | - | 9.852 | 0.050 | 0.051 | - |

(continued)

| | A solution | | | | B solution | | | | C solution | | | | Ester-compound-containing composition | | | |
|---|---|---|---|---|---|---|---|---|---|---|---|---|---|---|---|---|
| | BMA [g] | Component A | | | BMA [g] | Component B | | | BMA [g] | Component C | | | BMA [g] | A solution [g] | B solution [g] | C solution [g] |
| | | Compound name | Addition amount [g] | Contained amount [ppm by mass] | | Compound name | Addition amount [g] | Contained amount [ppm by mass] | | Compound name | Addition amount [g] | Contained amount [ppm by mass] | | | | |
| Example 13 | 10.001 | 2-Methyl-1-butanol | 0.020 | 2016 | 10.00 1 | DMTBP | 0.020 | 2036 | - | - | - | - | 9.436 | 0.501 | 0.051 | - |
| Example 14 | 10.001 | 2-Methyl-1-butanol | 0.020 | 2016 | 10.00 1 | DMTBP | 0.020 | 2036 | - | - | - | - | 4.930 | 5.000 | 0.050 | - |
| Example 15 | 10.001 | 2-Methyl-1-butanol | 0.020 | 2016 | - | - | - | - | - | - | - | - | 9.901 | 0.050 | - | - |

[Table 2]

| | A solution | | | | B solution | | | | C solution | | | | Ester-compound-containing composition | | | |
|---|---|---|---|---|---|---|---|---|---|---|---|---|---|---|---|---|
| | | Component A | | | | Component B | | | | Component C | | | | | | |
| | BMA [g] | Compound name | Addition amount [g] | Contained amount [ppm by mass] | BMA [g] | Compound name | Addition amount [g] | Contained amount [ppm by mass] | BMA [g] | Compound name | Addition amount [g] | Contained amount [ppm by mass] | BMA [g] | A solution [g] | B solution [g] | C solution [g] |
| Example 16 | 10.001 | 1-Pentanol | 0.021 | 2046 | 10.001 | DMTBP | 0.020 | 2036 | - | - | - | - | 9.851 | 0.050 | 0.050 | - |
| Example 17 | 10.001 | 1-Pentanol | 0.021 | 2046 | 10.001 | DMTBP | 0.020 | 2036 | - | - | - | - | 9.435 | 0.502 | 0.050 | - |
| Example 18 | 10.001 | 1-Pentanol | 0.021 | 2046 | 10.001 | DMTBP | 0.020 | 2036 | - | - | - | - | 4.931 | 5.004 | 0.050 | - |
| Example 19 | 10.001 | 1-Pentanol | 0.021 | 2046 | - | - | - | - | - | - | - | - | 9.901 | 0.050 | - | - |
| Example 20 | 10.001 | 2,4-Dimethyl-1,3-dioxane | 0.020 | 2026 | 10.002 | DMTBP | 0.020 | 1996 | - | - | - | - | 9.851 | 0.051 | 0.051 | - |
| Example 21 | 10.001 | 2,4-Dimethyl-1,3-dioxane | 0.020 | 2026 | 10.002 | DMTBP | 0.020 | 1996 | - | - | - | - | 9.436 | 0.501 | 0.050 | - |
| Example 22 | 10.001 | 2,4-Dimethyl-1,3-dioxane | 0.020 | 2026 | 10.002 | DMTBP | 0.020 | 1996 | - | - | - | - | 4.931 | 5.001 | 0.051 | - |
| Example 23 | 10.003 | 2,4-Dimethyl-1,3-dioxane | 1.000 | 90912 | 10.002 | DMTBP | 0.020 | 1996 | - | - | - | - | 8.851 | 1.100 | 0.050 | - |
| Example 24 | 10.003 | 2,4-Dimethyl-1,3-dioxane | 1.000 | 90912 | 10.002 | DMTBP | 0.020 | 1996 | - | - | - | - | 5.001 | 4.951 | 0.050 | - |
| Example 25 | 10.001 | 2,4-Dimethyl-1,3-dioxane | 0.020 | 2026 | 10.002 | DMTBP | 0.020 | 1996 | - | - | - | - | 9.436 | 0.051 | 0.500 | - |

(continued)

| | A solution | | | | B solution | | | | C solution | | | | Ester-compound-containing composition | | | |
|---|---|---|---|---|---|---|---|---|---|---|---|---|---|---|---|---|
| | BMA [g] | Component A | | | BMA [g] | Component B | | | BMA [g] | Component C | | | BMA [g] | A solution [g] | B solution [g] | C solution [g] |
| | | Compound name | Addition amount [g] | Contained amount [ppm by mass] | | Compound name | Addition amount [g] | Contained amount [ppm by mass] | | Compound name | Addition amount [g] | Contained amount [ppm by mass] | | | | |
| Example 26 | 10.001 | 2,4-Dimethyl-1,3-dioxane | 0.020 | 2026 | - | - | - | - | - | - | - | - | 9.902 | 0.050 | - | - |
| Example 27 | 10.001 | 2,4-Dimethyl-1,3-dioxane | 0.020 | 2026 | 10.005 | MEIIQ | 0.020 | 2015 | - | - | - | - | 9.851 | 0.051 | 0.050 | - |
| Example 28 | 10.001 | 2,4-Dimethyl-1,3-dioxane | 0.020 | 2026 | 10.003 | IPPDA | 0.020 | 2025 | - | - | - | - | 9.852 | 0.050 | 0.050 | - |
| Example 29 | 10.001 | 2,4-Dimethyl-1,3-dioxane | 0.020 | 2026 | 10.001 | PTZ | 0.020 | 2006 | - | - | - | - | 9.852 | 0.051 | 0.050 | - |
| Example 30 | 10.001 | 2,4-Dimethyl-1,3-dioxane | 0.020 | 2026 | 10.009 | AMX | 0.020 | 2004 | - | - | - | - | 9.852 | 0.051 | 0.051 | - |

[Table 3]

| | A solution | | | | B solution | | | | C solution | | | | Ester-compound-containing composition | | | |
|---|---|---|---|---|---|---|---|---|---|---|---|---|---|---|---|---|
| | BMA [g] | Component A | | | BMA [g] | Component B | | | BMA [g] | Component C | | | BMA [g] | A solution [g] | B solution [g] | C solution [g] |
| | | Compound name | Addition amount [g] | Contained amount [ppm by mass] | | Compound name | Addition amount [g] | Contained amount [ppm by mass] | | Compound name | Addition amount [g] | Contained amount [ppm by mass] | | | | |
| Example 31 | 10.001 | 2.2-Dibutoxypropane | 0.020 | 1996 | 9.813 | DMTBP | 0.020 | 2075 | - | - | - | - | 9.851 | 0.050 | 0.051 | - |
| Example 32 | 10.001 | 2.2-Dibutoxypropane | 0.020 | 1996 | 9.813 | DMTBP | 0.020 | 2075 | - | - | - | - | 9.436 | 0.501 | 0.050 | |
| Example 33 | 10.001 | 2,2-Dibutoxypropane | 0.020 | 1996 | 9.813 | DMTBP | 0.020 | 2075 | - | - | - | - | 5.129 | 5.020 | 0.050 | - |
| Example 34 | 10.001 | 2,2-Dibutoxypropane | 0.020 | 1996 | 10.000 | DMTBP | 0.020 | 1996 | - | - | - | - | 9.456 | 0.050 | 0.503 | - |
| Example 35 | 10.001 | 2,2-Dibutoxypropane | 0.020 | 1996 | 10.000 | DMTBP | 0.020 | 1996 | - | - | - | - | 4.936 | 0.050 | 5.002 | - |
| Example 36 | 10.001 | 2,2-Dibutoxypropane | 0.020 | 1996 | 9.986 | DMTBP | 1.002 | 91176 | - | - | - | - | 8.850 | 0.050 | 1.104 | - |
| Example 37 | 10.001 | 2,2-Dibutoxypropane | 0.020 | 1996 | - | - | - | - | - | - | - | - | 9.902 | 0.050 | - | - |
| Comparative Example 1 | 10.001 | 2.2-Dibutoxypropane | 0.020 | 1996 | 10.000 | DMTBP | 0.020 | 1996 | - | - | - | - | 9.402 | 0.051 | 0.051 | 0.500 |
| Comparative Example 2 | - | - | - | - | 9.924 | DMTBP | 0.020 | 2041 | 9.982 | 1-Butanol | 0.020 | 2010 | 9.436 | - | 0.050 | 0.504 |
| Comparative Example 3 | - | - | - | - | 9.924 | DMTBP | 0.020 | 2041 | 9.982 | 1-Butanol | 0.020 | 2010 | 4.930 | - | 0.050 | 5.001 |
| Comparative Example 4 | - | - | - | - | 9.924 | DMTBP | 0.020 | 2041 | 10.002 | 1-Butanol | 0.995 | 90478 | 8.851 | - | 0.051 | 1.101 |
| Comparative Example 5 | - | - | - | - | - | - | - | - | - | - | - | - | 10.005 | - | - | - |
| Comparative Example 6 | - | - | - | - | 12.198 | DMTBP | 0.024 | 1996 | - | - | - | - | 9.904 | - | 0.053 | - |

(continued)

| | A solution | | | | B solution | | | | C solution | | | | Ester-compound-containing composition | | | |
|---|---|---|---|---|---|---|---|---|---|---|---|---|---|---|---|---|
| | BMA [g] | Component A | | | BMA [g] | Component B | | | BMA [g] | Component C | | | BMA [g] | A solution [g] | B solution [g] | C solution [g] |
| | | Compound name | Addition amount [g] | Contained amount [ppm by mass] | | Compound name | Addition amount [g] | Contained amount [ppm by mass] | | Compound name | Addition amount [g] | Contained amount [ppm by mass] | | | | |
| Comparative Example 7 | 10.145 | Butyric acid | 0.020 | 1938 | 9.850 | DMTBP | 0.020 | 2057 | 0.000 | 1-Butanol | 1.000 | 1000000 | 9.469 | 0.051 | 0.056 | 0.506 |
| Comparative Example 8 | 10.001 | Butyraldehyde | 0.020 | 1996 | 10.002 | DMTBP | 0.020 | 2016 | 0.000 | 1-Butanol | 1.000 | 1000000 | 9.401 | 0.050 | 0.051 | 0.503 |

Abbreviations in Tables 1 to 3 have the following meanings.

- BMA: butyl methacrylate
- DMTBP: 2,4-dimethyl-6-t-butylphenol
- MEHQ: 4-methoxyphenol
- IPPDA: 4-isopropylaminodiphenylamine
- PTZ: phenothiazine
- HO-TEMPO: 4-hydroxy-2,2,6,6-tetramethyl-piperidine-N-oxyl
- AMX: 4-acetamido-2,2,6,6-tetramethyl-piperidine-N-oxyl

Tables 4 and 5 show the measurement results of the generated amounts of the BMA dimer and the butyl pyruvate in Examples 1 to 37 and Comparative Examples 1 to 8.

[Table 4]

| | Formulation of ester-compound-containing composition (actually measured value) | | | | | | | Analysis result of ester-compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generated amount of BMA dimer [mg/L] | Generated amount of methyl pyruvate [mg/L] |
| Example 1 | 99.88 | 2-Methyl-1-butanol | 10 | DMTBP | 10 | - | - | 1.69 | 2.76 |
| Example 2 | 99.88 | 2-Methyl-1-butanol | 102 | DMTBP | 10 | - | - | 1.70 | 3.30 |
| Example 3 | 99.78 | 2-Methyl-1-butanol | 1018 | DMTBP | 10 | - | - | 1.02 | 2.96 |
| Example 4 | 98.89 | 2-Methyl-1-butanol | 10009 | DMTBP | 10 | - | - | 0.96 | 0.49 |
| Example 5 | 99.88 | 2-Methyl-1-butanol | 10 | DMTBP | 102 | - | - | 1.67 | 2.32 |
| Example 6 | 99.78 | 2-Methyl-1-butanol | 10 | DMTBP | 1023 | - | - | 2.27 | 1.98 |
| Example 7 | 99.89 | 2-Methyl-1-butanol | 10 | - | - | - | - | 2.34 | 3.49 |
| Example 8 | 99.88 | 2-Methyl-1-butanol | 10 | MEHQ | 10 | - | - | 0.68 | 3.57 |
| Example 9 | 99.88 | 2-Methyl-1-butanol | 10 | IPPDA | 10 | - | - | 0.87 | 1.60 |
| Example 10 | 99.88 | 2-Methyl-1-butanol | 10 | PTZ | 10 | - | - | 0.65 | 3.15 |
| Example 11 | 99.88 | 2-Methyl-1-butanol | 10 | HO-TEMPO | 10 | - | - | 1.11 | 1.60 |
| Example 12 | 99.88 | 2-Methyl-1-butanol | 10 | DMTBP | 10 | - | - | 0.14 | 2.89 |
| Example 13 | 99.88 | 2-Methyl-1-butanol | 101 | DMTBP | 10 | - | - | 0.48 | 2.63 |
| Example 14 | 99.79 | 2-Methyl-1-butanol | 1010 | DMTBP | 10 | - | - | 0.80 | 2.94 |
| Example 15 | 99.89 | 2-Methyl-1-butanol | 10 | - | - | - | - | 0.07 | 2.94 |
| Example 16 | 99.88 | 1-Pentanol | 10 | DMTBP | 10 | - | - | 0.12 | 2.75 |
| Example 17 | 99.88 | 1-Pentanol | 103 | DMTBP | 10 | - | - | 0.10 | 2.71 |
| Example 18 | 99.78 | 1-Pentanol | 1025 | DMTBP | 10 | - | - | 1.20 | 3.45 |
| Example 19 | 99.89 | 1-Pentanol | 10 | - | - | - | - | 0.03 | 2.77 |
| Example 20 | 99.89 | 2,4-Dimethyl-1,3-dioxane | 10 | DMTBP | 10 | - | - | 1.51 | 1.53 |
| Example 21 | 99.88 | 2,4-Dimethyl-1,3-dioxane | 102 | DMTBP | 10 | - | - | 1.60 | 1.34 |
| Example 22 | 99.79 | 2,4-Dimethyl-1,3-dioxane | 1015 | DMTBP | 10 | - | - | 2.02 | 1.23 |

(continued)

| | Formulation of ester-compound-containing composition (actually measured value) | | | | | | | Analysis result of ester-compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generated amount of BMA dimer [mg/L] | Generated amount of methyl pyruvate [mg/L] |
| Example 23 | 98.89 | 2,4-Dimethyl-1,3-dioxane | 10000 | DMTBP | 10 | - | - | 2.63 | 1.59 |

[Table 5]

| | Formulation of ester-compound-containing composition (actually measured value) | | | | | | | Analysis result of ester-compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generated amount of BMA dimer [mg/L] | Generated amount of methyl pyruvate [mg/L] |
| Example 24 | 95.39 | 2,4-Dimethyl-1,3-dioxane | 45000 | DMTBP | 10 | - | - | 1.86 | 2.63 |
| Example 25 | 99.88 | 2,4-Dimethyl-1,3-dioxane | 10 | DMTBP | 100 | - | - | 3.08 | 1.24 |
| Example 26 | 99.89 | 2,4-Dimethyl-1,3-dioxane | 10 | - | - | - | - | 3.47 | 1.37 |
| Example 27 | 99.89 | 2,4-Dimethyl-1,3-dioxane | 10 | MEHQ | 10 | - | - | 2.42 | 1.32 |
| Example 28 | 99.89 | 2,4-Dimethyl-1,3-dioxane | 10 | IPPDA | 10 | - | - | 2.45 | 1.15 |
| Example 29 | 99.89 | 2,4-Dimethyl-1,3-dioxane | 10 | PTZ | 10 | - | - | 2.78 | 1.31 |
| Example 30 | 99.89 | 2,4-Dimethyl-1,3-dioxane | 10 | AMX | 10 | - | - | 2.37 | 1.21 |
| Example 31 | 99.89 | 2,2-Dibutoxypropane | 10 | DMTBP | 11 | - | - | 0.39 | 3.49 |
| Example 32 | 99.88 | 2,2-Dibutoxypropane | 100 | DMTBP | 10 | - | - | 1.82 | 3.42 |
| Example 33 | 99.79 | 2,2-Dibutoxypropane | 982 | DMTBP | 10 | - | - | 2.00 | 4.02 |
| Example 34 | 99.88 | 2,2-Dibutoxypropane | 10 | DMTBP | 100 | - | - | 1.45 | 3.10 |
| Example 35 | 99.79 | 2,2-Dibutoxypropane | 10 | DMTBP | 1000 | - | - | 2.72 | 2.12 |
| Example 36 | 98.88 | 2,2-Dibutoxypropane | 10 | DMTBP | 10058 | - | - | 2.66 | 2.34 |

(continued)

| | Formulation of ester-compound-containing composition (actually measured value) | | | | | | | Analysis result of ester-compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generated amount of BMA dimer [mg/L] | Generated amount of methyl pyruvate [mg/L] |
| Example 37 | 99.89 | 2,2-Dibutoxypropane | 10 | - | - | - | - | 2.23 | 3.57 |
| Comparative Example 1 | 94.89 | 2,2-Dibutoxypropane | 10 | DMTBP | 10 | 1-Butanol | 49979 | 5.01 | 6.05 |
| Comparative Example 2 | 99.88 | - | - | DMTBP | 10 | 1-Butanol | 101 | 3.51 | 4.38 |
| Comparative Example 3 | 99.79 | - | - | DMTBP | 10 | 1-Butanol | 1007 | 3.84 | 4.64 |
| Comparative Example 4 | 98.89 | - | - | DMTBP | 10 | 1-Butanol | 9955 | 3.50 | 6.32 |
| Comparative Example 5 | 99.91 | - | - | - | - | - | - | 5.74 | 7.63 |
| Comparative Example 6 | 99.89 | - | - | DMTBP | 11 | - | - | 5.38 | 6.27 |
| Comparative Example 7 | 94.88 | Butyric acid | 10 | DMTBP | 12 | 1-Butanol | 50208 | 4.18 | 15.04 |
| Comparative Example 8 | 94.87 | Butyraldehyde | 10 | DMTBP | 10 | 1-Butanol | 50317 | 2.95 | 10.27 |

As shown in Table 4 and Table 5, in the ester-compound-containing compositions of Examples 1 to 37 in which the contained amount of BMA was 95% by mass or more and component A was contained, the generated amounts of the BMA dimer and the butyl pyruvate after storage were smaller than those of the ester-compound-containing compositions of Comparative Examples 1 to 8 in which the contained amount of BMA was less than 95% by mass or component A was not contained.

[Example 38]

0.020 g of butyraldehyde as a component A was added to 10.001 g of butyl methacrylate (BMA, manufactured by Tokyo Chemical Industry Co., Ltd., moisture content: 120 ppm by mass) as an ester compound (I) to prepare an A solution.

In addition, 0.020 g of 2,4-dimethyl-6-t-butylphenol as a component B was added to 10.002 g of BMA (moisture content: 120 ppm by mass) as an ester compound (I) to prepare a B solution.

Next, 0.050 g of the A solution and 0.052 g of the B solution were added to 9.845 g of BMA (moisture content: 120 ppm by mass) as an ester compound (I) to prepare an ester-compound-containing composition.

The obtained ester-compound-containing composition was stored at 25°C for 14 days under white LED illumination, a contained amount (mg/L) of BMA dimer was determined by the GC-MS measurement using the absolute calibration curve method, and the amount of change (generated amount) before and after storage was calculated.

[Examples 39 to 55 and Comparative Examples 5 and 6]

An ester-compound-containing composition was prepared in the same manner as in Example 38, except that the formulations of the A solution, the B solution, and the ester-compound-containing composition were changed as shown in Table 6, and the generated amount of BMA dimer was obtained.

[Table 6]

| | A solution | | | | B solution | | | | Ester-compound-containing composition | | |
|---|---|---|---|---|---|---|---|---|---|---|---|
| | BMA [g] | Component A | | | BMA [g] | Component B | | | BMA [g] | A solution [g] | B solution [g] |
| | | Compound name | Addition amount [g] | Contained amount [ppm by mass] | | Compound name | Addition amount [g] | Contained amount [ppm by mass] | | | |
| Example 38 | 10.001 | Butyraldehyde | 0.020 | 1996 | 10.002 | DMTBP | 0.020 | 2016 | 9.845 | 0.050 | 0.052 |
| Example 39 | 10.001 | Butyraldehyde | 0.020 | 1996 | 10.003 | DMTBP | 0.020 | 2016 | 9.436 | 0.501 | 0.052 |
| Example 40 | 10001 | Butyraldehyde | 0.020 | 1996 | 10.002 | DMTBP | 0.020 | 2016 | 4.933 | 4.999 | 0.050 |
| Example 41 | 10.006 | Butyraldehyde | 1.002 | 90987 | 10.002 | DMTBP | 0.020 | 2016 | 8.850 | 1.104 | 0.051 |
| Example 42 | 10.006 | Butyraldehyde | 1.002 | 90987 | 10.002 | DMTBP | 0.020 | 2016 | 4.999 | 4.950 | 0.050 |
| Example 43 | 10.001 | Butyraldehyde | 0.020 | 1996 | 10.002 | DMTBP | 0.020 | 2016 | 9.436 | 0.051 | 0.501 |
| Example 44 | 10.001 | Butyraldehyde | 0.020 | 1996 | 10.002 | DMTBP | 0.020 | 2016 | 4.932 | 0.050 | 5.000 |
| Example 45 | 10.001 | Butyraldehyde | 0.020 | 1996 | 10.004 | DMTBP | 1.002 | 91041 | 8.850 | 0.050 | 1.101 |
| Example 46 | 10001 | Butyraldehyde | 0.020 | 1996 | 10.000 | - | - | - | 9.902 | 0.050 | - |
| Example 47 | 10.145 | Butyric acid | 0.020 | 1938 | 9.850 | DMTBP | 0.020 | 2057 | 9.843 | 0.051 | 0.053 |
| Example 48 | 9.746 | Butyric acid | 0.021 | 2099 | 9.850 | DMTBP | 0.020 | 2057 | 9.843 | 0.051 | 0.053 |
| Example 49 | 10.145 | Butyric acid | 0.020 | 1938 | 9.850 | DMTBP | 0.020 | 2057 | 9.441 | 0.503 | 0.050 |
| Example 50 | 10.145 | Butyric acid | 0.020 | 1938 | 9.850 | DMTBP | 0.020 | 2057 | 4.927 | 5.019 | 0.050 |
| Example 51 | 10.025 | Butyric acid | 0.998 | 90507 | 9.850 | DMTBP | 0.020 | 2057 | 8.849 | 1.142 | 0.050 |
| Example 52 | 10.025 | Butyric acid | 0.998 | 90507 | 9.850 | DMTBP | 0.020 | 2057 | 5.051 | 4.962 | 0.049 |
| Example 53 | 10.145 | Butyric acid | 0.020 | 1938 | 9.850 | DMTBP | 0.020 | 2057 | 9.433 | 0.050 | 0.503 |
| Example 54 | 10.145 | Butyric acid | 0.020 | 1938 | 9.850 | DMTBP | 0.020 | 2057 | 4.946 | 0.049 | 5.003 |
| Example 55 | 10.145 | Butyric acid | 0.020 | 1938 | 10.000 | - | - | - | 9.892 | 0.051 | - |
| Comparative Example 5 | - | - | - | - | - | - | - | - | 10.005 | - | - |
| Comparative Example 6 | - | - | - | - | 12.198 | DMTBP | 0.024 | 1996 | 9.904 | - | 0.053 |

Abbreviations in Table 6 have the following meanings.

- BMA: butyl methacrylate
- DMTBP: 2,4-dimethyl-6-t-butylphenol

Table 7 shows the measurement results of the generated amount of the BMA dimer in Examples 38 to 55 and Comparative Examples 5 and 6.

[Table 7]

| | Formulation of ester-compound-containing composition (actually measured value) | | | | | Analysis result of ester-compound-containing composition after storage |
|---|---|---|---|---|---|---|
| | BMA [% by mass] | Component A compound name | Component A [ppm by mass] | Component B compound name | Component B [ppm by mass] | Generated amount of BMA [mg/L] |
| Example 38 | 99.89 | Butyraldehyde | 10 | DMTBP | 10 | 3.63 |
| Example 39 | 99.89 | Butyraldehyde | 100 | DMTBP | 10 | 3.44 |
| Example 40 | 99.80 | Butyraldehyde | 1000 | DMTBP | 10 | 2.95 |
| Example 41 | 98.89 | Butyraldehyde | 10036 | DMTBP | 10 | 3.19 |
| Example 42 | 95.40 | Butyraldehyde | 45038 | DMTBP | 10 | 2.38 |
| Example 43 | 99.89 | Butyraldehyde | 10 | DMTBP | 101 | 3.32 |
| Example 44 | 99.80 | Butyraldehyde | 10 | DMTBP | 1010 | 3.15 |
| Example 45 | 98.90 | Butyraldehyde | 10 | DMTBP | 10018 | 3.66 |
| Example 46 | 99.90 | Butyraldehyde | 10 | - | - | 3.67 |
| Example 47 | 99.90 | Butyric acid | 10 | DMTBP | 11 | 4.33 |
| Example 48 | 99.90 | Butyric acid | 11 | DMTBP | 11 | 3.64 |
| Example 49 | 99.89 | Butyric acid | 98 | DMTBP | 10 | 4.02 |
| Example 50 | 99.80 | Butyric acid | 973 | DMTBP | 10 | 3.90 |
| Example 51 | 98.87 | Butyric acid | 10296 | DMTBP | 10 | 4.20 |
| Example 52 | 95.44 | Butyric acid | 44635 | DMTBP | 10 | 3.32 |
| Example 53 | 99.89 | Butyric acid | 10 | DMTBP | 103 | 4.20 |
| Example 54 | 99.79 | Butyric acid | 10 | DMTBP | 1029 | 4.42 |
| Example 55 | 99.90 | Butyric acid | 10 | - | - | 4.55 |
| Comparative Example 5 | 99.91 | - | - | - | - | 5.74 |
| Comparative Example 6 | 99.89 | - | - | DMTBP | 11 | 5.38 |

As shown in Table 7, in the ester-compound-containing compositions of Examples 38 to 55 in which component A was contained, the generated amount of the BMA dimer after storage was smaller than that of the ester-compound-containing compositions of Comparative Examples 5 and 6 in which component A was not contained.

[Example 56]

0.020 g of 2-methyl-1-butanol as a component A was added to 10.001 g of isobutyl methacrylate (IBMA,

manufactured by Tokyo Chemical Industry Co., Ltd., moisture content: 110 ppm by mass) as an ester compound (I) to prepare an A solution.

In addition, 0.020 g of 2,4-dimethyl-6-t-butylphenol as a component B was added to 10.084 g of IBMA (moisture content: 110 ppm by mass) as an ester compound (I) to prepare a B solution.

Next, 0.050 g of the A solution and 0.051 g of the B solution were added to 9.850 g of IBMA (moisture content: 110 ppm by mass) as an ester compound (I) to prepare an ester-compound-containing composition.

The obtained ester-compound-containing composition was stored at 70°C for 7 hours under white LED illumination, contained amounts (mg/L) of IBMA dimer and isobutyl pyruvate were determined by the GC-MS measurement using the absolute calibration curve method, and the amount of change (generated amount) before and after storage was calculated.

[Examples 57 to 75 and Comparative Examples 9 to 12]

An ester-compound-containing composition was prepared in the same manner as in Example 56, except that the formulations of the A solution, the B solution, a C solution (solution containing a component C), and the ester-compound-containing composition were changed as shown in Tables 8 and 9, and the generated amounts of the IBMA dimer and the isobutyl pyruvate were obtained.

[Table 8]

| | A solution | | | | B solution | | | | C solution | | | | Ester-compound-containing composition | | | |
|---|---|---|---|---|---|---|---|---|---|---|---|---|---|---|---|---|
| | IBMA [g] | Component A | | | IBMA [g] | Component B | | | IBMA [g] | Component C | | | IBMA [g] | A solution [g] | B solution [g] | C solution [g] |
| | | Compound name | Addition amount [g] | Contained amount [ppm by mass] | | Compound name | Addition amount [g] | Contained amount [ppm by mass] | | Compound name | Addition amount [g] | Contained amount [ppm by mass] | | | | |
| Example 56 | 10.001 | 2-Methyl-1-butanol | 0.020 | 1996 | 10.084 | DMTBP | 0.020 | 1999 | - | - | - | - | 9.850 | 0.050 | 0.051 | - |
| Example 57 | 10.001 | 2-Methyl-1-butanol | 0.020 | 1996 | 10.084 | DMTBP | 0.020 | 1999 | - | - | - | - | 9.435 | 0.501 | 0.050 | - |
| Example 58 | 10.001 | 2-Methyl-1-butanol | 0.020 | 1996 | 10.084 | DMTBP | 0.020 | 1999 | - | - | - | - | 4.931 | 5.006 | 0.050 | - |
| Example 59 | 10.002 | 2-Methyl-1-butanol | 1.000 | 90918 | 10.084 | DMTBP | 0.020 | 1999 | - | - | - | - | 8.877 | 1.131 | 0.051 | - |
| Example 60 | 10.002 | 2-Methyl-1-butanol | 1.000 | 90918 | 10.084 | DMTBP | 0.020 | 1999 | - | - | - | - | 5.001 | 4.952 | 0.050 | - |
| Example 61 | 10.001 | 2-Methyl-1-butanol | 0.020 | 1996 | 10.084 | DMTBP | 0.020 | 1999 | - | - | - | - | 9.436 | 0.050 | 0.503 | - |
| Example 62 | 10.001 | 2-Methyl-1-butanol | 0.020 | 1996 | 10.084 | DMTBP | 0.020 | 1999 | - | - | - | - | 4.930 | 0.050 | 5.001 | - |
| Example 63 | 10.001 | 2-Methyl-1-butanol | 0.020 | 1996 | - | - | - | - | - | - | - | - | 9902 | 0.050 | - | - |
| Example 64 | 10.001 | 2-Methyl-1-butanol | 0.021 | 2046 | 10.003 | DMTBP | 0.020 | 2005 | - | - | - | - | 9.851 | 0.052 | 0.051 | - |
| Example 65 | 10.001 | 2-Methyl-1-butanol | 0.021 | 2046 | 10.003 | DMTBP | 0.020 | 2005 | - | - | - | - | 9.449 | 0.502 | 0.051 | - |
| Example 66 | 10.001 | 2-Methyl-1-butanol | 0.021 | 2046 | 10.003 | DMTBP | 0.020 | 2005 | - | - | - | - | 4.934 | 5.001 | 0.050 | - |
| Example 67 | 10.001 | 2-Methyl-1-butanol | 1.000 | 90926 | 10.003 | DMTBP | 0.020 | 2005 | - | - | - | - | 8.850 | 1.102 | 0.051 | - |

[Table 9]

| | A solution | | | | B solution | | | | C solution | | | | Ester-compound-containing composition | | | |
|---|---|---|---|---|---|---|---|---|---|---|---|---|---|---|---|---|
| | IBMA [g] | Component A | | | IBMA [g] | Component B | | | IBMA [g] | Component C | | | IBMA [g] | A solution [g] | B solution [g] | C solution [g] |
| | | Compound name | Addition amount [g] | Contained amount [ppm by mass] | | Compound name | Addition amount [g] | Contained amount [ppm by mass] | | Compound name | Addition amount [g] | Contained amount [ppm by mass] | | | | |
| Example 68 | 10.001 | 2-Methyl-1-butanol | 1.000 | 90926 | 10.003 | DMTBP | 0.020 | 2005 | - | - | - | - | 5.000 | 4.951 | 0.050 | - |
| Example 69 | 10.001 | 2-Methyl-1-butanol | 0.021 | 2046 | - | - | - | - | - | - | - | - | 9.901 | 0.050 | - | - |
| Example 70 | 10.000 | 2-Methyl-1-butanol | 0.020 | 2026 | 10.003 | DMTBP | 0.020 | 2005 | - | - | - | - | 9.854 | 0.051 | 0.051 | - |
| Example 71 | 10.000 | 2-Methyl-1-butanol | 0.020 | 2026 | 10.003 | DMTBP | 0.020 | 2005 | - | - | - | - | 9.436 | 0.502 | 0.050 | - |
| Example 72 | 10.000 | 2-Methyl-1-butanol | 0.020 | 2026 | 10.003 | DMTBP | 0.020 | 2005 | - | - | - | - | 4.931 | 5.002 | 0.051 | - |
| Example 73 | 10.000 | 2-Methyl-1-butanol | 1.000 | 90906 | 10.003 | DMTBP | 0.020 | 2005 | - | - | - | - | 8.850 | 1.102 | 0.051 | - |
| Example 74 | 10.000 | 2-Methyl-1-butanol | 1.000 | 90906 | 10.003 | DMTBP | 0.020 | 2005 | - | - | - | - | 5.001 | 4.951 | 0.051 | - |
| Example 75 | 10.000 | 2-Methyl-1-butanol | 0.020 | 2026 | - | - | - | - | - | - | - | | 9.906 | 0.051 | - | - |
| Comparative Example 9 | - | - | - | - | 10.084 | DMTBP | 0.020 | 1999 | 10.000 | Isobutanol | 1.000 | 90907 | 8.851 | - | 0.050 | 1.101 |
| Comparative Example 10 | - | - | - | - | 10.084 | DMTBP | 0.020 | 1999 | 10.000 | Isobutanol | 1.000 | 90907 | 5.001 | - | 0.050 | 4.952 |
| Comparative Example 11 | - | - | - | - | - | - | - | - | - | - | - | - | 10.005 | - | - | - |
| Comparative Example 12 | - | - | - | - | 12.494 | DMTBP | 0.025 | 1997 | - | - | - | - | 9.905 | - | 0.051 | - |

Abbreviations in Tables 8 and 9 have the following meanings.

- IBMA: isobutyl methacrylate
- DMTBP: 2,4-dimethyl-6-t-butylphenol

Table 10 shows the measurement results of the generated amounts of the IBMA dimer and the isobutyl pyruvate in Examples 56 to 75 and Comparative Examples 9 to 12.

[Table 10]

| | Formulation of ester-compound-containing composition (actually measured value) | | | | | | | Analysis result of ester-compound-containing composition after storage | |
|---|---|---|---|---|---|---|---|---|---|
| | IBMA [% by mass] | Component A compound name | Component A [ppm by mass] | Component B compound name | Component B [ppm by mass] | Component C compound name | Component C [ppm by mass] | Generated amount of IBMA dimer [mg/L] | Generated amount of isobutyl pyruvate [mg/L] |
| Example 56 | 99.61 | 2-Methyl-1-butanol | 10 | DMTBP | 10 | - | - | 12.08 | 0.17 |
| Example 57 | 99.61 | 2-Methyl-1-butanol | 100 | DMTBP | 10 | - | - | 11.03 | 0.19 |
| Example 58 | 99.52 | 2-Methyl-1-butanol | 1000 | DMTBP | 10 | - | - | 11.46 | 0.29 |
| Example 59 | 98.60 | 2-Methyl-1-butanol | 10221 | DMTBP | 10 | - | - | 11.60 | 0.32 |
| Example 60 | 95.13 | 2-Methyl-1-butanol | 45009 | DMTBP | 10 | - | - | 10.93 | 0.55 |
| Example 61 | 99.61 | 2-Methyl-1-butanol | 10 | DMTBP | 101 | - | - | 12.48 | 0.20 |
| Example 62 | 99.52 | 2-Methyl-1-butanol | 10 | DMTBP | 1002 | - | - | 11.55 | 0.33 |
| Example 63 | 99.62 | 2-Methyl-1-butanol | 10 | - | - | - | - | 10.43 | 0.21 |
| Example 64 | 99.61 | 2-Methyl-1-butanol | 11 | DMTBP | 10 | - | - | 10.32 | 0.16 |
| Example 65 | 99.60 | 2-Methyl-1-butanol | 103 | DMTBP | 10 | - | - | 11.07 | 0.30 |
| Example 66 | 99.51 | 2-Methyl-1-butanol | 1025 | DMTBP | 10 | - | - | 11.10 | 0.19 |
| Example 67 | 98.62 | 2-Methyl-1-butanol | 10017 | DMTBP | 10 | - | - | 9.97 | 0.33 |
| Example 68 | 95.13 | 2-Methyl-1-butanol | 45011 | DMTBP | 10 | - | - | 9.99 | 0.44 |

(continued)

| | Formulation of ester-compound-containing composition (actually measured value) | | | | | | | Analysis result of ester-compound-containing composition after storage | |
|---|---|---|---|---|---|---|---|---|---|
| | IBMA [% by mass] | Component A compound name | Component A [ppm by mass] | Component B compound name | Component B [ppm by mass] | Component C compound name | Component C [ppm by mass] | Generated amount of IBMA dimer [mg/L] | Generated amount of isobutyl pyruvate [mg/L] |
| Example 69 | 99.62 | 2-Methyl-1-butanol | 10 | - | - | - | - | 10.38 | 0.19 |
| Example 70 | 99.61 | 2-Methyl-1-butanol | 10 | DMTBP | 10 | - | - | 11.59 | 0.21 |
| Example 71 | 99.60 | 2-Methyl-1-butanol | 102 | DMTBP | 10 | - | - | 8.15 | 0.09 |
| Example 72 | 99.51 | 2-Methyl-1-butanol | 1015 | DMTBP | 10 | - | - | 7.87 | 0.16 |
| Example 73 | 98.62 | 2-Methyl-1-butanol | 10017 | DMTBP | 10 | - | - | 9.60 | 0.24 |
| Example 74 | 95.13 | 2-Methyl-1-butanol | 44996 | DMTBP | 10 | - | - | 12.41 | 0.28 |
| Example 75 | 99.62 | 2-Methyl-1-butanol | 10 | - | - | - | - | 12.66 | 0.10 |
| Comparative Example 9 | 98.62 | - | - | DMTBP | 10 | Isobutanol | 10005 | 13.51 | 1.07 |
| Comparative Example 10 | 95.13 | - | - | DMTBP | 10 | Isobutanol | 45003 | 12.27 | 3.17 |
| Comparative Example 11 | 99.69 | - | - | - | - | - | - | 13.68 | 0.83 |
| Comparative Example 12 | 99.69 | - | - | DMTBP | 10 | - | - | 12.77 | 0.69 |

As shown in Table 10, in the ester-compound-containing compositions of Examples 56 to 75 in which component A was contained, the generated amounts of the IBMA dimer and the isobutyl pyruvate after storage were smaller than those of the ester-compound-containing compositions of Comparative Examples 9 to 12 in which component A was not contained.

INDUSTRIAL APPLICABILITY

According to the present invention, an ester-compound-containing composition which can be used as a raw material or the like of a (meth)acrylic polymer can be produced efficiently and can be stably stored for a long period of time, which is industrially useful.

## Claims

1. An ester-compound-containing composition, comprising:

one or more compounds selected from the group consisting of a compound represented by Formula (A1), a compound represented by Formula (A2), a compound represented by Formula (A3), a compound represented by Formula (A4), and a compound represented by Formula (A5); and
an ester compound (I) represented by Formula (1),
wherein a contained amount of the ester compound (I) is 95% to 99.99% by mass with respect to a total mass of the ester-compound-containing composition,

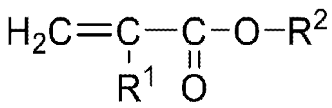
・・・(1)

(in Formula (1), $R^1$ is a hydrogen atom or a methyl group, and $R^2$ is a monovalent hydrocarbon group having 4 carbon atoms),

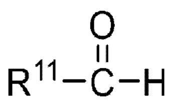
・・・(A1)

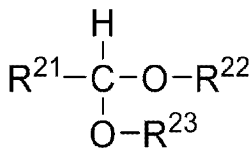
・・・(A2)

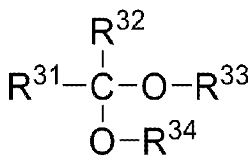
・・・(A3)

(in Formulae (A1) to (A3), $R^{11}$ and $R^{21}$ are each independently a linear or branched alkyl group having 1 to 20 carbon atoms, and $R^{22}$ and $R^{23}$, $R^{31}$ and $R^{32}$, and $R^{33}$ and $R^{34}$ may be each independently a linear or branched alkyl group having 1 to 20 carbon atoms, or may be a divalent group having 1 to 20 carbon atoms, which may have an ether bond in combination with each of the groups),

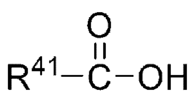
・・・(A4)

(in Formula (A4), $R^{41}$ is a linear or branched alkyl group having 1 to 20 carbon atoms),

$R^{51}$-OH ··· (A5)

(in Formula (A5), $R^{51}$ is a linear or branched alkyl group having 5 carbon atoms).

2. The ester-compound-containing composition according to Claim 1, wherein the ester compound (I) includes one or more selected from the group consisting of butyl (meth)acrylate and isobutyl (meth)acrylate.

3. The ester-compound-containing composition according to Claim 1, wherein the compounds represented by Formulae (A1) to (A5) are each at least one of butyraldehyde, 1,1-dibutoxybutane, 2,2-dibutoxypropane, 2,4-dimethyl-1,3-dioxane, 2,2,4-trimethyl-1,3-dioxane, 2-ethyl-4-methyl-1,3-dioxane, 1,3,5-trimethyl-2,4,6-trioxane, acetic acid, butyric acid, 1-pentyl alcohol, 2-methyl-1-butanol, or 3-methyl-1-butanol.

4. The ester-compound-containing composition according to Claim 1, wherein a total contained amount of the compounds represented by Formulae (A1) to (A5) is 1 ppm by mass or more and 1500 ppm by mass or less.

5. The ester-compound-containing composition according to Claim 1, further comprising: a component B: a polymerization inhibitor.

6. The ester-compound-containing composition according to Claim 5, wherein component B is at least one polymerization inhibitor selected from the group consisting of a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

7. The ester-compound-containing composition according to Claim 5, wherein component B is at least one polymerization inhibitor selected from the group consisting of a phenol-based compound, an N-oxyl-based compound, an amine-based compound, a phosphorus-containing compound, and a sulfur-containing compound.

8. The ester-compound-containing composition according to Claim 5, wherein component B is at least one polymerization inhibitor selected from the group consisting of hydroquinone, 4-methoxyphenol, 2,4-dimethyl-6-t-butylphenol, and 2,6-di-t-butyl-4-methylphenol.

9. The ester-compound-containing composition according to Claim 1, wherein the contained amount of the ester compound (I) is 99% to 99.99% by mass.

10. The ester-compound-containing composition according to Claim 1, wherein the ester-compound-containing composition is stored for 1 day or longer.

11. A method for producing an ester-compound-containing composition which contains an ester compound (I) represented by Formula (1), the production method comprising:

performing an ester exchange reaction between methyl (meth)acrylate and a monoalcohol (II) having 4 carbon atoms in the presence of one or more compounds selected from the group consisting of a compound represented by Formula (A1), a compound represented by Formula (A2), a compound represented by Formula (A3), a compound represented by Formula (A4), and a compound represented by Formula (A5),

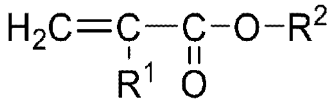 ··· (1)

(in Formula (1), $R^1$ is a hydrogen atom or a methyl group, and $R^2$ is a monovalent hydrocarbon group having 4 carbon atoms),

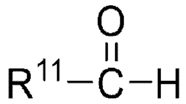 ・・・(A1)

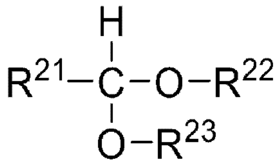 ・・・(A2)

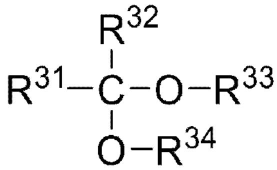 ・・・(A3)

(in Formulae (A1) to (A3), $R^{11}$ and $R^{21}$ are each independently a linear or branched alkyl group having 1 to 20 carbon atoms, and $R^{22}$ and $R^{23}$, $R^{31}$ and $R^{32}$, and $R^{33}$ and $R^{34}$ may be each independently a linear or branched alkyl group having 1 to 20 carbon atoms, or may be a divalent group having 1 to 20 carbon atoms, which may have an ether bond in combination with each of the groups),

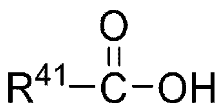 ・・・(A4)

(in Formula (A4), $R^{41}$ is a linear or branched alkyl group having 1 to 20 carbon atoms),

$R^{51}$-OH ··· (A5)

(in Formula (A5), $R^{51}$ is a linear or branched alkyl group having 5 carbon atoms).

**12.** The method for producing an ester-compound-containing composition according to Claim 11,
wherein the monoalcohol (II) is at least one of n-butanol or isobutanol.

**13.** The method for producing an ester-compound-containing composition according to Claim 11,
wherein the compounds represented by Formulae (A1) to (A5) include at least one of butyraldehyde, 1,1-dibutoxybutane, 2,2-dibutoxypropane, 2,4-dimethyl-1,3-dioxane, 2,2,4-trimethyl-1,3-dioxane, 2-ethyl-4-methyl-1,3-dioxane, 1,3,5-trimethyl-2,4,6-trioxane, acetic acid, butyric acid, 1-pentyl alcohol, 2-methyl-1-butanol, or 3-methyl-1-butanol.

**14.** A polymerizable composition for producing a (meth)acrylic polymer, comprising:
the ester-compound-containing composition according to any one of Claims 1 to 10.

**15.** The polymerizable composition according to Claim 14, further comprising:
a monomer copolymerizable with the ester compound (1).

**16.** A (meth)acrylic polymer obtained by polymerizing the polymerizable composition according to Claim 14.

**17.** A (meth)acrylic polymer obtained by polymerizing the polymerizable composition according to Claim 15.

**18.** A method for producing a (meth)acrylic polymer, comprising:
polymerizing the polymerizable composition according to Claim 14.

**19.** A method for producing a (meth)acrylic polymer, comprising:
polymerizing the polymerizable composition according to Claim 15.

**INTERNATIONAL SEARCH REPORT**

International application No.
**PCT/JP2023/039202**

**A. CLASSIFICATION OF SUBJECT MATTER**

***C08F 20/18***(2006.01)i; ***C07C 67/03***(2006.01)i; ***C07C 67/62***(2006.01)i; ***C07C 69/54***(2006.01)i
FI: C08F20/18; C07C67/03; C07C69/54 Z CSP; C07C67/62

According to International Patent Classification (IPC) or to both national classification and IPC

**B. FIELDS SEARCHED**

Minimum documentation searched (classification system followed by classification symbols)

C08F20/18; C07C67/03; C07C67/62; C07C69/54

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2005-315930 A (NIPPON ZEON CO., LTD.) 10 November 2005 (2005-11-10) paragraphs [0021]-[0022], example 3 | 16-17 |
| A | entire text | 1-15, 18-19 |
| A | JP 2007-063171 A (UBE IND., LTD.) 15 March 2007 (2007-03-15) entire text | 1-19 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 December 2023** | **09 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/039202**

| Patent document cited in search report | Publication date (day/month/year) | Patent family member(s) | Publication date (day/month/year) |
|---|---|---|---|
| JP 2005-315930 A | 10 November 2005 | (Family: none) | |
| JP 2007-063171 A | 15 March 2007 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022174445 A
- JP 2022174450 A
- JP 2022174455 A
- JP 2009274986 A